# EUROPEAN PATENT SPECIFICATION

(11) **EP 3 911 761 B1**
(45) Date of publication and mention of the grant of the patent: **30.10.2024**
(21) Application number: 20741208.1
(22) Date of filing: 20.01.2020
(51) Int. Cl.: C12Q 1/6876, C12Q 1/6874, C12Q 1/6811, C12Q 1/6883, C12Q 1/6888

(54) **DNA METHYLATION MEASUREMENT FOR MAMMALS BASED ON CONSERVED LOCI**
DNA-METHYLIERUNGSMESSUNG FÜR SÄUGETIERE BASIEREND AUF KONSERVIERTEN LOCI
MESURE DE MÉTHYLATION D'ADN POUR DES MAMMIFÈRES SUR LA BASE DE LOCI CONSERVÉS

(30) Priority: 18.01.2019 US 201962794364 P
(43) Date of publication of application: 24.11.2021
(73) Proprietor: The Regents of the University of California, Oakland CA 94607-5200 (US); Illumina, Inc., San Diego, CA 92122 (US)
(72) Inventor: HORVATH, Stefan, Los Angeles, CA 90049 (US); ERNST, Jason, Los Angeles, CA 90024 (US); ARNESON, Adriana, Cristina, San Francisco, CA 94107 (US); BARNES, Bret, Carlsbad, CA 92008 (US)
(74) Representative: Murgitroyd & Company
(86) International application number: PCT/US2020/014251
(87) International publication number: WO 2020/150705

(56) References cited:
- WO-A1-2009/021141
- WONG NICHOLAS C ET AL: "Exploring the utility of human DNA methylation arrays for profiling mouse genomic DNA", GENOMICS, ACADEMIC PRESS, SAN DIEGO, US, vol. 102, no. 1, 29 April 2013 (2013-04-29), pages 38 - 46, XP028560874, ISSN: 0888-7543, DOI: 10.1016/J.YGENO.2013.04.014
- JA-RANG LEE ET AL: "Successful application of human-based methyl capture sequencing for methylome analysis in non-human primate models", BMC GENOMICS, BIOMED CENTRAL LTD, LONDON, UK, vol. 19, no. 1, 18 April 2018 (2018-04-18), pages 1 - 12, XP021255537, DOI: 10.1186/S12864-018-4666-1
- CHRISTINE COULDREY ET AL: "Assessing DNA methylation levels in animals: choosing the right tool for the job", ANIMAL GENETICS, BLACKWELL SCIENTIFIC PUBLICATIONS, LONDON, GB, vol. 45, 2 July 2014 (2014-07-02), pages 15 - 24, XP071536178, ISSN: 0268-9146, DOI: 10.1111/AGE.12186
- QU ET AL.: "Evolutionary expansion of DNA hypomethylation in the mammalian germline genome", GENOME RES, vol. 28, no. 2, February 2018 (2018-02-01), pages 145 - 158, XP055725670
- WONG ET AL.: "Exploring the utility of human DNA methylation arrays for profiling mouse genomic DNA", GENOMICS, vol. 102, no. 1, July 2013 (2013-07-01), pages 38 - 46, XP028560874, DOI: 10.1016/j.ygeno.2013.04.014
- MCLAIN ET AL.: "The evolution of CpG density and lifespan in conserved primate and mammalian promoters", AGING, vol. 10, no. 4, 14 April 2018 (2018-04-14), XP055725675
- BIBIKOVA ET AL.: "High density DNA methylation array with single CpG site resolution", GENOMICS, vol. 98, no. 4, October 2011 (2011-10-01), pages 288 - 295, XP028304083, DOI: 10.1016/j.ygeno.2011.07.007

## Description

### TECHNICAL FIELD

The invention relates to methods and materials for examining methylation of genomic DNA in mammals.

### BACKGROUND OF THE INVENTION

DNA methylation by the attachment of a methyl group to cytosines is one of the most widely studies epigenetic modifications, due to its implications in regulating gene expression across many biological processes (1,2). In humans, DNA methylation levels can be used to accurately predict an individual's age, as well as age across tissues and cell types (3).

The two most widely used technologies for obtaining DNA methylation levels are bisulfite sequencing and microarray-based methylation chips. Whole genome bisulfite sequencing is an expensive assay, causing reduced representation bisulfite sequencing (RRBS) to become the prevalent sequencing approach. RRBS effectively queries only a small number of nucleotides on the genome but still provides a genome wide methylation profile. However, the sequencing depth required even for RRBS can still drive up costs. Due to this, for human samples, array chips containing an increasing number of polynucleotide probes have been the most reliable and widely used technology (4-6).

The first human methylation chip (ILLUMINA INFINIUM 27K) was introduced over ten years ago. However, no analogous chip has been presented for other non human mammalian species, a delay which may reflect the fact that it is not economical to design conventional methylation chips for non-human mammals. For example, the development and use of conventional species-specific methylation chips/arrays could hinder cross species comparisons as the measurement platforms are different. In view of this, conventional species-specific methylation chips may be sub-optimal for cross-species comparisons. Consequently, there is a need for methods and materials useful for observing methylation and phenomena associated with methylation (e.g. aging) across a wide variety of mammalian species.

Wong et al., "Exploring the utility of human DNA methylation arrays for profiling mouse genomic DNA", Genomics, vol. 102, no. 1, 29 April 2013, Ja-Rang et al., "Successful application of human-based methyl capture sequencing for methylome analysis in non-human primate models", BMC Genomics, vol. 19, no. 1, 18 April 2018, pages 1-12, Couldrey et al., "Assessing DNA methylation levels in animals: choosing the right tool for the job", Animal Genetics, vol. 45, 2 July 2014, pages 15-24 are useful in understanding the background of the present invention.

### SUMMARY OF THE INVENTION

Provided herein are: two methods of making DNA methylation arrays, two DNA methylation arrays, a method of observing a methylation profile and a method of observing the effects of a test agent on genomic methylation. Accordingly, the present invention is as described in the claims.

Valuable information can be obtained from the study of methylation patterns in mammalian species other than those that are the typical focus of scientific studies (e.g. humans and mice). A problem in such studies however is the fact that it is technically challenging and expensive to develop methods and materials designed for observing methylation profiles in species that are rarely studied (e.g. naked mole-rats and killer whales). In this context, a single measurement platform that is useful to study all mammalian species would provide a solution that makes such endeavors much more efficient and cost effective. The invention disclosed herein provides this platform in the form of methods and materials that can be used to observe methylation and phenomena associated with methylation in a wide variety of mammalian species. As discussed below, one advantageous aspect of the invention is the identification and utilization of highly conserved segments of CpG methylation site containing DNA in the human genome, i.e. segments of the human genome that facilitate cross-species comparisons.

Disclosed herein are a number of embodiments. One embodiment is an algorithm termed "Conserved Methylation Array Probe Selector" (CMAPS). This algorithm is used to identify DNA sequences useful in embodiments of the invention such as DNA methylation arrays/chips by repurposing conventional degenerate base technologies that are used to tolerate within-human variation in a manner that allows polynucleotides to tolerate cross-species mutations. In embodiments of the invention, the CMAPS algorithm performs a comprehensive sequence search to obtain a maximal number of species that can be targeted using a single probe for a CpG in the human genome, based on a multiple sequence alignment. The CMAPS algorithm then ranks all the sequences/probes and chooses a final set so that such sequences can be used to query a large number of mammalian species at varied genomic positions based on external annotations of exons, CpG islands and hyper versus hypo methylated regions.

The CMAPS algorithm can be used, for example, to facilitate the design of embodiments of the invention, including DNA methylation arrays (e.g. arrays of polynucleotides disposed on a matrix such as a bead or chip). One such embodiment of the invention is a DNA methylation array comprising a plurality of polynucleotides coupled to a matrix, wherein the plurality of polynucleotides are selected by: (a) performing a polynucleotide sequence alignment comparing a human genome with a plurality of non-human mammalian genomes to identify polynucleotide sequences in the human genome comprising CpG methylation sites that are homologous to polynucleotide sequences within genomes of non-human mammalian species comprising CpG methylation sites; (b) ranking the polynucleotide sequences in the human genome identified in (a), wherein the ranking criteria comprises sequence homology to polynucleotide sequences in genomes of non-human mammalian species and then (c) using the ranking in (b) to select a plurality of polynucleotides in the human genome that cross hybridize to a plurality of polynucleotide sequences in the genomes of non-human mammalian species. Other illustrative ranking criteria can comprise for example, identifying those CpG containing polynucleotide sequences that function in the greatest number of different mammalian species; and/or identifying those CpG containing polynucleotide sequences that have been characterized as being significant in other epigenetic biomarker studies (e.g. human aging studies).

Typically in these embodiments of the invention, the plurality of human genomic polynucleotide sequences are selected to have not more than a 3 base pair mismatch with polynucleotide sequences in genomes of non-human mammalian species. Optionally, the ranking sequence alignment compares human genomic sequences with genomic sequences of at least 10, 20, 30, 40 or more non-human mammalian species, and/or comprises comparisons of human genomic polynucleotide sequences to genomic polynucleotide sequences in evolutionarily distant species such as non-placental mammalian species as well as placental mammalian species. In certain embodiments of the invention, the DNA methylation chip comprises at least 10,000, 20,000 or 30,000 unique polynucleotides coupled to the matrix. Typically, the polypeptides comprise about 60 nucleotides (e.g. 40-80 nucleotides) that are at least about 95% identical to a DNA segment of a nonhuman mammalian genome comprising a CpG methylation site (e.g. where 57 out of 60 nucleotides of a nonhuman mammalian genome are identical to a 60 nucleotide DNA segment of a human genome). In certain illustrative working embodiments of the invention disclosed herein, at least one polynucleotide within the plurality of polynucleotides is a polynucleotide having a sequence shown in Table 3.

A related embodiment of the invention is a DNA methylation array comprising a plurality of polynucleotide sequences coupled to a matrix, wherein the polynucleotides comprise a CpG motif (or its complement) at their terminal ends. These polynucleotides typically comprise sequences of about 60 nucleotides that exhibit an about 95% homology between a human genomic sequence and a genomic sequence of a non-human mammalian species (e.g. 57 out of 60 nucleotides). In certain embodiments of the invention, at least 2,000 polynucleotides within the plurality of polynucleotide sequences can hybridize to a 60 nucleotide segment in genomic polynucleotide sequences of a marsupial mammalian species (e.g. a wallaby species) with less than a 3 base pair mismatch; and/or at least 2,000 polynucleotides within the plurality of polynucleotide sequences can hybridize to a 60 nucleotide segment in genomic polynucleotide sequences of a monotreme mammalian species (e.g. a platypus) with less than a 3 base pair mismatch; and/or at least 2,000 polynucleotides within the plurality of polynucleotide sequences can hybridize to a 60 nucleotide segment in genomic polynucleotide sequences of a laurasiatherian mammalian species (e.g. a bat species) with less than a 3 base pair mismatch; and/or at least 2,000 polynucleotides within the plurality of polynucleotide sequences can hybridize to a 60 nucleotide segment in genomic polynucleotide sequences of a euarchontoglirian mammalian species (e.g. a rodent species) with less than a 3 base pair mismatch; and/or at least 2,000 polynucleotides within the plurality of polynucleotide sequences can hybridize to a 60 nucleotide segment in genomic polynucleotide sequences of a xenarthran mammalian species (e.g. an armadillo species) with less than a 3 base pair mismatch; and/or at least 2,000 polynucleotides within the plurality of polynucleotide sequences can hybridize to a 60 nucleotide segment in genomic polynucleotide sequences of a afrotherian mammalian species (e.g. a tenrec species) with less than a 3 base pair mismatch.

Another embodiment of the invention is a method of making a DNA methylation array comprising coupling a plurality of polynucleotides to a matrix. Typically in such embodiments of the invention, the plurality of polynucleotides each comprise a CpG motif (or its complement) and are polynucleotide sequences of about 60 nucleotides that exhibit an about 95% homology between a human genomic sequence and a non-human mammalian species (e.g. 57 out of 60 nucleotides). In typical embodiments of the invention, the DNA methylation array is designed so that it comprises at least 2,000 unique polynucleotide sequences that hybridize to a 60 nucleotide segment in genomic polynucleotide sequences of non-placental mammalian species as well as placental mammalian species with less than a 3 base pair mismatch. Typically, the plurality of polypeptides used to make the DNA methylation array are selected by: (i) performing a polynucleotide sequence alignment comparing a human genome with a plurality of non-human mammalian genomes to identify polynucleotide sequences in the human genome comprising CpG methylation sites that are homologous to polynucleotide sequences comprising CpG methylation sites within genomes of non-human mammalian species; (ii) ranking the polynucleotide sequences in the human genome identified in (a), wherein the ranking criteria comprises sequence homology to polynucleotide sequences in the genomes of non-human mammalian species; and then (iii) using the ranking in (b) to select a plurality of polynucleotides having CpG methylation sites that cross hybridize to a plurality of polynucleotide sequences having CpG methylation sites in the genomes of non-human mammalian species with not more than a 2, 3 or 4 base pair mismatch, so that the DNA methylation array is made.

Yet another embodiment of the invention is a method of observing a methylation profile in a non-human mammal comprising obtaining genomic DNA from the non-human mammal; and then observing cytosine methylation of a plurality CG loci in the genomic DNA using a DNA methylation array disclosed herein; so that a methylation profile in the non-human mammal is observed. Optionally this method includes comparing the CG locus methylation profile observed with the CG locus methylation profiles observed in genomic DNA derived from individuals in the non-human mammal species having known ages; and then correlating the CG locus methylation observed with the known ages of the non-human mammal species, so that information useful to determine the age of the non-human mammal is obtained. In typical embodiments of the invention, the DNA methylation array is used to observe methylation profiles in a plurality of non-human mammalian species. Significantly, embodiments of the invention further allow artisans to evaluate whether an intervention (e.g. exposure to a test agent) that affects DNA methylation levels in one species (e.g. mouse) also affects the corresponding DNA methylation levels in another species (e.g. human). In addition, the conserved sequences further allow artisans to develop epigenetic age estimators for different mammalian species (epigenetic clocks) based on highly conserved CpGs.

As discussed below, a working embodiment of the invention disclosed herein is termed the "HorvathMammalMethylChip40", and is a DNA methylation array disposed on a chip which contains roughly 38k unique human genomic polynucleotides coupled to a matrix as probes for complementary sequences. Among those, 36,000 polynucleotide probes query CpG sites in conserved regions of the mammalian genome, making this embodiment of the invention useful in studies across all mammalian species. In this embodiment of the invention, the remaining 2,000 probes were chosen due to their special interest in human epigenetic biomarker studies. As shown by the data presented in Table 2 below, the resulting DNA methylation chip is applicable to all mammals and hence drives down the cost per chip through economies of scale. Further, this chip embodiment is tailor-made for cross species comparisons.

### BRIEF DESCRIPTION OF THE DRAWINGS

**Figure 1.** Figure 1 provides graphed data showing CpG sites identified through the CMAPS algorithm target dense CpG islands due to the inclusion of Infinium I probes. The representation of the selected CpGs (blue) is similar to that of all CpGs in the human genome (red).
**Figure 2.** Figure 2 provides graphed data showing CpG sites identified through the CMAPS algorithm target both hyper and hypo methylated CpG sites. The histogram of methylation of the selected probes (red) is similar to that of all sites (blue) in the human genome.

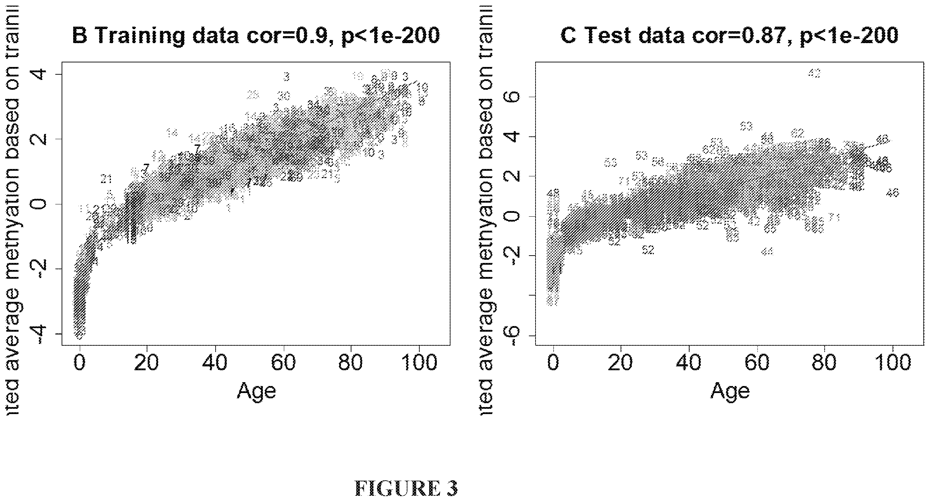

**Figure 3.** Figure 3 provides graphed data showing epigenetic aging clock based on 404 highly conserved CpGs from human methylation data. Left panel: the weighted average of the 404 epigenetic clock CpGs versus chronological age in the training data sets. The rate of change of the red curve can be interpreted as tick rate. Points are colored and labeled by data set as described in Horvath, S. DNA methylation age of human tissues and cell types. Genome Biol. 14, R115 (2013 ("Horvath 2013"). Right panel: analogous results for the test data sets. Only the test data lend themselves for independent validation.

### DETAILED DESCRIPTION OF THE INVENTION

In the description of embodiments, reference may be made to the accompanying figures which form a part hereof, and in which is shown by way of illustration a specific embodiment in which the invention may be practiced. Unless otherwise defined, all terms of art, notations and other scientific terms or terminology used herein are intended to have the meanings commonly understood by those of skill in the art to which this invention pertains. In some cases, terms with commonly understood meanings are defined herein for clarity and/or for ready reference, and the inclusion of such definitions herein should not necessarily be construed to represent a substantial difference over what is generally understood in the art.

U.S. Patent Publication 20150259742, U.S. Patent App. No. 15/025,185, titled "METHOD TO ESTIMATE THE AGE OF TISSUES AND CELL TYPES BASED ON EPIGENETIC MARKERS", filed by Stefan Horvath; U.S. Patent App. No. 14/119,145, titled "METHOD TO ESTIMATE AGE OF INDIVIDUAL BASED ON EPIGENETIC MARKERS IN BIOLOGICAL SAMPLE", filed by Eric Villain et al.; and Hannum et al. "Genome-Wide Methylation Profiles Reveal Quantitative Views Of Human Aging Rates." Molecular Cell. 2013; 49(2):359-367 and patent US2015/0259742, are relevant to the invention.

As noted above, embodiments of the invention disclosed herein include an algorithm for identifying highly conserved methylation probes (CMAPS) that are useful to observe genomic methylation patterns across a wide variety of mammalian species. The polynucleotide probe sequence information including specific nucleotides within each probe sequence is designed to be tolerable to specified variation. The polynucleotide probes identified by the CMAPS algorithm allow one to measure cytosine methylation levels in short stretches of DNA that are highly conserved across mammals using polynucleotide arrays such as those sold by ILLUMINA. Embodiments of the invention disclosed herein include gene chips comprising a plurality of human genomic sequences identified using the CMAPS algorithm.

As discussed below, an illustrative working embodiment of the invention that is disclosed herein is a gene chip comprising a set of 35,988 polynucleotide probes that allow one to assess cytosine DNA methylation levels in essentially all mammalian species. The CMAPS algorithm underlies the design of this custom ILLUMINA Infinium chip (HorvathMammalMethylChip40) which contains these roughly 38k polynucleotide probes. Among those, 36,000 probes query CpG sites in conserved regions of the human genome, making the chip applicable in all mammalian species. The remaining 2,000 probes were chosen due to their special interest in human epigenetic biomarker studies. This DNA methylation chip is useful for observing methylation profiles in all mammalian species and is therefore tailor-made for cross species comparisons.

Embodiments of the invention include, for example, methods of making a DNA methylation array comprising a plurality of polynucleotides coupled to a matrix such as a bead or a chip. Typically in these methods, the plurality of polynucleotides are selected by a method comprising: performing a polynucleotide sequence alignment comprising comparing a human genome with a plurality of non-human mammalian genomes to identify polynucleotide sequences in the human genome comprising CpG methylation sites that are homologous to polynucleotide sequences within genomes of non-human mammalian species comprising CpG methylation sites; ranking the polynucleotide sequences in the human genome identified in the polynucleotide sequence alignment, wherein the ranking criteria comprises sequence homology to polynucleotide sequences in genomes of non-human mammalian species; and using this ranking in to select a plurality of polynucleotides in the human genome that cross hybridize to a plurality of polynucleotide sequences in the genomes of non-human mammalian species; and then coupling selected sequences from to a matrix so as to form a DNA methylation array. In typical embodiments of the invention, the DNA methylation array comprises at least 30,000 unique polynucleotides coupled to the matrix.

In certain embodiments of the methods for making a DNA methylation array, the plurality of human genomic polynucleotide sequences are selected to have not more than a 3 base pair mismatch with polynucleotide sequences in genomes of non-human mammalian species. Typically, the plurality of polynucleotides are between 40-80 nucleotides in length. In some embodiments of the invention, the ranking of polynucleotide sequences comprises the step of homology comparisons to genomic polynucleotide sequences in non-placental mammalian species, and placental mammalian species in the Laurasiatheria, Euarchontoglires, Xenarthra and Afrotheria superordinal groups. Optionally, the sequence alignment compares human genomic sequences with genomic sequences of at least 10 non-human mammalian species.

In another illustrative embodiment of a method of making a DNA methylation array comprising a plurality of polynucleotides coupled to a matrix, the plurality of polynucleotides comprise a CpG motif, and comprise at least 2,000 polynucleotide sequences that hybridize to a 60 nucleotide segment in genomic polynucleotide sequences of a marsupial mammalian species, a monotreme mammalian species, a Laurasiatheria mammalian species, a Euarchontoglires mammalian species, a Xenarthra mammalian species and an Afrotheria mammalian species with less than a 3 base pair mismatch. Typically, the polynucleotide sequences are selected by performing a polynucleotide sequence alignment comparing a human genome with a plurality of non-human mammalian genomes to identify polynucleotide sequences in the human genome comprising CpG methylation sites that are homologous to polynucleotide sequences comprising CpG methylation sites within genomes of non-human mammalian species; ranking the polynucleotide sequences in the human genome identified in (a), wherein the ranking criteria comprises a degree of sequence homology to polynucleotide sequences in the genomes of non-human mammalian species; using the rankings to select a plurality of polynucleotides having CpG methylation sites that cross hybridize to a plurality of polynucleotide sequences having CpG methylation sites in the genomes of non-human mammalian species with not more than a 3 base pair mismatch; and then coupling selected sequences from step (b) to a matrix so as to form a DNA methylation array so that the DNA methylation array is made.

Embodiments of the invention include a DNA methylation array made by a method disclosed herein. In certain embodiments of the invention, at least 1, 10, 100 or more polynucleotides within the plurality of polynucleotides is a polynucleotide having a sequence shown in Table 3. For example, embodiments of the invention include a DNA methylation array comprising a plurality of polynucleotide sequences coupled to a matrix, wherein the polynucleotides comprise at least 60 nucleotides and a CpG motif at their terminal ends; the polynucleotides comprise polynucleotide sequences present in a human genome; and at least 2,000 polynucleotides within the plurality of polynucleotide sequences can hybridize to a 60 nucleotide segment in genomic polynucleotide sequences of a marsupial mammalian species with less than a 3 base pair mismatch; at least 2,000 polynucleotides within the plurality of polynucleotide sequences can hybridize to a 60 nucleotide segment in genomic polynucleotide sequences of a monotreme mammalian species with less than a 3 base pair mismatch; at least 2,000 polynucleotides within the plurality of polynucleotide sequences can hybridize to a 60 nucleotide segment in genomic polynucleotide sequences of a Laurasiatheria mammalian species with less than a 3 base pair mismatch; at least 2,000 polynucleotides within the plurality of polynucleotide sequences can hybridize to a 60 nucleotide segment in genomic polynucleotide sequences of a Euarchontoglires mammalian species with less than a 3 base pair mismatch; at least 2,000 polynucleotides within the plurality of polynucleotide sequences can hybridize to a 60 nucleotide segment in genomic polynucleotide sequences of a Xenarthra mammalian species with less than a 3 base pair mismatch; and at least 2,000 polynucleotides within the plurality of polynucleotide sequences can hybridize to a 60 nucleotide segment in genomic polynucleotide sequences of a Afrotheria mammalian species with less than a 3 base pair mismatch. In certain embodiments, the marsupial mammalian species is a Wallaby species; and/or the monotreme mammalian species is a Platypus species; and/or the Laurasiatheria mammalian species is a bat species; and/or the Euarchontoglires mammalian species is a rodent species; and/or the Xenarthra mammalian species is an armadillo species; and/or the Afrotheria mammalian species is a tenrec species.

Another embodiment of the invention is a method of observing a methylation profile in a non-human mammal comprising obtaining genomic DNA from the non-human mammal; observing cytosine methylation of a plurality CG loci in the genomic DNA using a DNA methylation array disclosed herein; so that a methylation profile in the non-human mammal is observed. Optionally these methods also include comparing the CG locus methylation observed in the method to the CG locus methylation observed in genomic DNA derived from individuals in the non-human mammal species having known ages; and then correlating the CG locus methylation observed in (b) with the known ages of the non-human mammal species; so that information useful to determine the age of the non-human mammal is obtained. Typically in these embodiments, methylation is observed by a process comprising treatment of genomic DNA from the population of cells from the mammals with bisulfite to transform unmethylated cytosines of CpG dinucleotides in the genomic DNA to uracil; the DNA methylation array is used to observe methylation profiles in a plurality of non-human mammalian species; and/or genomic DNA is amplified by a polymerase chain reaction process.

Yet another embodiment of the invention is methods of observing the effects of a test agent (a compound having a molecular weight less than 3,000, 2,000, 1,000 or 500 g/mol, for example rapamycin) on genomic methylation associated epigenetic aging of mammalian cells (e.g. human primary keratinocytes). Typically these methods comprise combining the test agent with mammalian cells; observing methylation status of methylation markers in genomic DNA from the mammalian cells using a DNA methylation array of disclosed herein; and then comparing these observations with observations of the methylation status in genomic DNA from control mammalian cells not exposed to the test agent such that effects of the test agent on genomic methylation associated epigenetic aging in the mammalian cells is observed (e.g. whether or not the test agent decreases or increases genomic methylation patterns that are associated with epigenetic aging). Optionally in these methods, a plurality of test agents are combined with the mammalian cells. In certain embodiments of these methods, polynucleotides are coupled to a matrix, methylation is observed by a process comprising treatment of genomic DNA from the population of cells from the mammals with bisulfite to transform unmethylated cytosines of CpG dinucleotides in the genomic DNA to uracil; and/or genomic DNA is amplified by a polymerase chain reaction process.

Further aspects and embodiments of the invention are discussed in the following sections.

### FURTHER ILLUSTRATIVE ASPECTS AND EMBODIMENTS OF THE INVENTION

DNA methylation refers to chemical modifications of the DNA molecule. Technological platforms such as the ILLUMINA Infinium microarray or DNA sequencing-based methods have been found to lead to highly robust and reproducible measurements of the DNA methylation levels in humans. There are more than 28 million CpG loci in the human genome. Consequently, certain loci are given unique identifiers such as those cataloged in the ILLUMINA CpG loci database and used in Table 3 (see, e.g. Technical Note: Epigenetics, CpG Loci Identification ILLUMINA Inc. 2010). Certain illustrative CG locus designation identifiers and sequences are used herein. Such sequences can further be characterized using one or more of the genomic databases that are readily available to artisans in this technology such as the UCSC Genome Browser, an on-line, and downloadable, genome browser hosted by the University of California, Santa Cruz (UCSC).

The term "epigenetic" as used herein means relating to, being, or involving a chemical modification of the DNA molecule. Epigenetic factors include the addition or removal of a methyl group which results in changes of the DNA methylation levels.

The term "polynucleotide" as used herein may include any polymer or oligomer of pyrimidine and purine bases, preferably cytosine, thymine, and uracil, and adenine and guanine, respectively. The present invention contemplates any deoxyribonucleotide, ribonucleotide or peptide nucleic acid component, and any chemical variants thereof, such as methylated, hydroxymethylated or glucosylated forms of these bases, and the like. The polymers or oligomers may be heterogeneous or homogeneous in composition, and may be isolated from naturally-occurring sources or may be artificially or synthetically produced. In addition, the nucleic acids may be DNA or RNA, or a mixture thereof, and may exist permanently or transitionally in single-stranded or double-stranded form, including homoduplex, heteroduplex, and hybrid states.

The term "methylation marker" as used herein refers to a CpG position that is potentially methylated. Methylation typically occurs in a CpG containing nucleic acid. The CpG containing nucleic acid may be present in, e.g., in a CpG island, a CpG doublet, a promoter, an intron, or an exon of gene. For instance, in the genetic regions provided herein the potential methylation sites encompass the promoter/enhancer regions of the indicated genes. Thus, the regions can begin upstream of a gene promoter and extend downstream into the transcribed region.

The phrase "selectively measuring" as used herein refers to methods wherein only a finite number of methylation marker or genes (comprising methylation markers) are measured rather than assaying essentially all potential methylation marker (or genes) in a genome. For example, in some aspects, "selectively measuring" methylation markers or genes comprising such markers can refer to measuring no less (or no more) than 100, 75, 50, 25, 10 or 5 different methylation markers or genes comprising methylation markers.

DNA methylation of the methylation markers (or markers close to them) can be measured using various approaches, which range from commercial array platforms (e.g. from ILLUMINA) to sequencing approaches of individual genes. This includes standard lab techniques or array platforms. A variety of methods for detecting methylation status or patterns have been described in, for example U.S. Pat. Nos. 6,214,556, 5,786,146, 6,017,704, 6,265,171, 6,200,756, 6,251,594, 5,912,147, 6,331,393, 6,605,432, and 6,300,071 and US Patent Application Publication Nos. 20030148327, 20030148326, 20030143606, 20030082609 and 20050009059. Other array-based methods of methylation analysis are disclosed in U.S. patent application publication No. 11/058,566. For a review of some methylation detection methods, see, Oakeley, E. J., Pharmacology & Therapeutics 84:389-400 (1999). Available methods include but are not limited to: reverse-phase HPLC, thin-layer chromatography, Sssl methyltransferases with incorporation of labeled methyl groups, the chloracetaldehyde reaction, differentially sensitive restriction enzymes, hydrazine or permanganate treatment (m5C is cleaved by permanganate treatment but not by hydrazine treatment), sodium bisulfite, combined bisulphate-restriction analysis, and methylation sensitive single nucleotide primer extension. The ILLUMINA method takes advantage of sequences flanking a CpG locus to generate a unique CpG locus cluster ID with a similar strategy as NCBI's refSNP IDs (rs#) in dbSNP (see, e.g. Technical Note: Epigenetics, CpG Loci Identification ILLUMINA Inc. 2010).

The methylation levels of a subset of the DNA methylation markers disclosed herein are assayed (e.g. using an ILLUMINA DNA methylation array or using a PCR protocol involving relevant primers). To quantify the methylation level, one can follow the standard protocol described by ILLUMINA to calculate the beta value of methylation, which equals the fraction of methylated cytosines in that location. The invention can also be applied to any other approach for quantifying DNA methylation at locations near the genes as disclosed herein. DNA methylation can be quantified using many currently available assays.

In certain embodiments of the invention, the genomic DNA is hybridized to a complimentary sequence (e.g. a synthetic polynucleotide sequence) that is coupled to a matrix (e.g. one disposed within a microarray). Optionally, the genomic DNA is transformed from its natural state via amplification by a polymerase chain reaction process. For example, prior to or concurrent with hybridization to an array, the sample may be amplified by a variety of mechanisms, some of which may employ PCR. See, for example, PCR Technology: Principles and Applications for DNA Amplification (Ed. H. A. Erlich, Freeman Press, NY, N.Y., 1992); PCR Protocols: A Guide to Methods and Applications (Eds. Innis, et al., Academic Press, San Diego, Calif., 1990); Mattila et al., Nucleic Acids Res. 19, 4967 (1991); Eckert et al., PCR Methods and Applications 1, 17 (1991); PCR (Eds. McPherson et al., IRL Press, Oxford); and U.S. Pat. Nos. 4,683,202, 4,683,195, 4,800,159, 4,965,188, and 5,333,675. The sample may be amplified on the array. See, for example, U.S. Pat. No. 6,300,070.

Embodiments of the invention can include a variety of art accepted technical processes. For example, in certain embodiments of the invention, a bisulfite conversion process is performed so that cytosine residues in the genomic DNA are transformed to uracil, while 5-methylcytosine residues in the genomic DNA are not transformed to uracil. Kits for DNA bisulfite modification are commercially available from, for example, MethylEasyTM (Human Genetic SignaturesTM) and CpGenomeTM Modification Kit (ChemiconTM). See also, WO04096825A1, which describes bisulfite modification methods and Olek et al. Nuc. Acids Res. 24:5064-6 (1994), which discloses methods of performing bisulfite treatment and subsequent amplification. Bisulfite treatment allows the methylation status of cytosines to be detected by a variety of methods. For example, any method that may be used to detect a SNP may be used, for examples, see Syvanen, Nature Rev. Gen. 2:930-942 (2001). Methods such as single base extension (SBE) may be used or hybridization of sequence specific probes similar to allele specific hybridization methods. In another aspect the Molecular Inversion Probe (MIP) assay may be used.

Many techniques exist for measuring DNA methylation levels in a single species. For measuring methylation in human DNA, one can use the human ILLUMINA Infinium arrays to measure DNA methylation levels in human DNA samples. A recent paper (Needhamsen et al., BMC Bioinformatics, BMC series - 2017, 18:486) has shown that it is possible to use the EPIC chip for methylation measurements in mouse, but only ~19K out of the 850K probes on the EPIC chip are useful in mouse. Species that are more distant from human are likely to have even fewer useful probes on the EPIC chip, pointing to the need for a platform that can be used in non-human mammals.

An alternative to chips/arrays for measuring DNA methylation is bisulfite sequencing (see, e.g. Meissner et al., Nucleic Acids Research, Volume 33, Issue 18, 1 January 2005, Pages 5868-5877), which applies to all mammalian species, but is not established to be as quantitatively reliable. Array technology is particularly valuable for developing highly robust epigenetic biomarkers of aging and development. The current invention provides an algorithm for selecting probes and the results of this algorithm for identifying (non-natural) nucleotide sequences which can be used on methylation arrays/chips that apply to all mammals. We have demonstrated that highly conserved sequences lend themselves for building highly accurate epigenetic aging clocks (see, e.g. U.S. Patent App. No. 15/025,185, titled "METHOD TO ESTIMATE THE AGE OF TISSUES AND CELL TYPES BASED ON EPIGENETIC MARKERS").

The first human methylation chip (ILLUMINA Infinium 27K) was introduced over ten years ago but no analogous chip has been presented for other species. This delay may reflect the fact that it is not economical to design a methylation chip for non-human species. Even if costs were no impediment, the development of species-specific arrays could hinder cross species comparisons as the measurement platforms would be different. As noted above, to address these challenges, we developed an algorithm, Conserved Methylation Array Probe Selector (CMAPS), which repurposes the degenerate base technology used to tolerate within-human variation to tolerate cross-species mutations. CMAPS performs a greedy search to obtain a maximal number of species that can be targeted using a probe for any CpG in the human genome, based on a multiple sequence alignment. CMAPS was used to design almost 36,000 probes querying CpG sites in conserved regions of the human genome, making the chip directly applicable to mammalian species and thus facilitating cross species comparisons. To obtain such a large number of probes for a large number of species, CMAPS ranks all the probes and chooses a final set such that each Infinium array can query a large number of mammalian species and varied genomic positions based on external annotations of exons, CpG islands and hyper versus hypo methylated regions. To enhance the utility of the chip in human studies, we also added about 2,000 probes that are of particular interest in human biomarker studies. In the following, we describe the CMAPS algorithm and the properties of the resulting chip (HorvathMammalMethylChip40)

### ILLUMINA Infinium probes

Currently, methylation arrays produced by ILLUMINA can contain two types of probes: Infinium 1 and Infinium 2, with the latter being newer technology requiring only one bead to query a CG, while the former requires two beads.

For the design and development of the MammalMethyl40 chip, we leveraged a list of all the human CG sites, which can be interrogated using one or both of these probes. There are two variants of each of the two probes, depending on whether the probe is designed on the forward versus reverse genomic strand. The probes allow for up to 3 degenerate bases, which can tolerate variation in the sequence being interrogated. The number of degenerate bases tolerated is a function of the design score of the probe computed by ILLUMINA, and the number of underlying CpGs in the case of Infinium 2 probes **(Table 1).**

In order to be able to query a certain CpG site, an oligonucleotide probe has to be synthesized on the array containing the 60 base pairs either upstream or downstream of the CpG site. Degenerate base technology allows for a CpG site to be interrogated by a probe even if an individual happens to have variants in the neighboring region that cause mismatches with the synthesized probe **(Methods).** We developed the CMAPS algorithm, which repurposes this technology to design degenerate bases for each human probe, so that the probe can now tolerate mutations and hybridize to DNA from other species as well. The CMAPS algorithm was applied to a 100-way alignment of 99 other species to the human genome and provides the ability to pick mutations within the rules specified by the underlying array technology, which is the Infinium technology in this particular case. However, the algorithm can take as input any multiple sequence alignment with any reference genome, along with a set of design considerations and provide conserved probes and degenerate base selections within those rules.

### Determining an initial set of 60,000probes

For each CG site in the human genome we selected the Infinium 1 probe out of the options that covered the most species based on the algorithm described above, and analogously for Infinium 2. We first included all Infinium 2 probes that were targeting the mm10 mouse genome, such that the chip is guaranteed to be useful for one of the most widely used model organisms. We then sorted the CpG sites in descending order of the number of species covered with the Infinium 2 probe, and added all the probes that weren't already selected due to targeting mm10, for a total of up to 53,000 probes. We then ranked the probes on the ILLUMINA EPIC array in descending order of the number of species they can target using the degenerate bases picked by the CMAPS algorithm, and selected an additional 3,000 probes that had not already been picked based on the earlier criteria. Lastly, we sorted the CpG sites in descending order of number of species they can target and picked the top 4,000 Infinium 1 probes that targeted CpG sites that had not already been included. The Infinium 1 probes were selected to allow us to query more CG dense regions, as the underlying CpG count of an Infinium 1 probe does not count against the number of SNVs permitted. This gave us a total of 60,000 probes.

### Filtering probes based on mappability

Since probes on the array are only 60 base pairs long, they run the risk of mapping to multiple locations in the genome, which results in a confounded signal coming from multiple CpG sites. This issue can be compounded by the fact that each of our probes can have up to 2^(# of degenerate bases) variants. For 16 quality genomes we computed for each probe how many of its variants map uniquely in that genome. We then filtered probes down by asking that all variants of a probe have to map uniquely in at least 80% of the species they were designed to target, or the probe has to target at least 40 species. This reduced the set of working probes to the final set of 35,988 probes.

### Properties of the custom chip

The *HorvathMammalMethylChip40* profiles fewer than 40k probes (hence the ending "40").

Two thousand out of 38k probes were selected based on their utility for human biomarker studies. These CpGs, which were previously implemented in human ILLUMINA Infinium arrays (EPIC, 450K, 27K) were selected due to their relevance for estimating age, blood cell counts, or the proportion of neurons in brain tissue.

The remaining 35,988 probes were chosen to assess cytosine DNA methylation levels in a wide variety of evolutionarily distinct mammalian species. Toward this end, the CMAPS algorithm was employed to identify highly conserved CpGs across 50 mammalian species: 33,493 Infinium II probes and 2,496 Infinium I probes. Not all probes on the array are expected to work for all species, but rather each probe is designed to cover a certain subset of species, such that overall all species have a high number of probes. The particular subset of species for each probe is provided in the chip manifest file. Out of the 50 mammalian species observed, 46 of them have more than 10,000 probes on the array, and 36 have more than 20,000 probes **(Table 2).**

### Chromosomal context

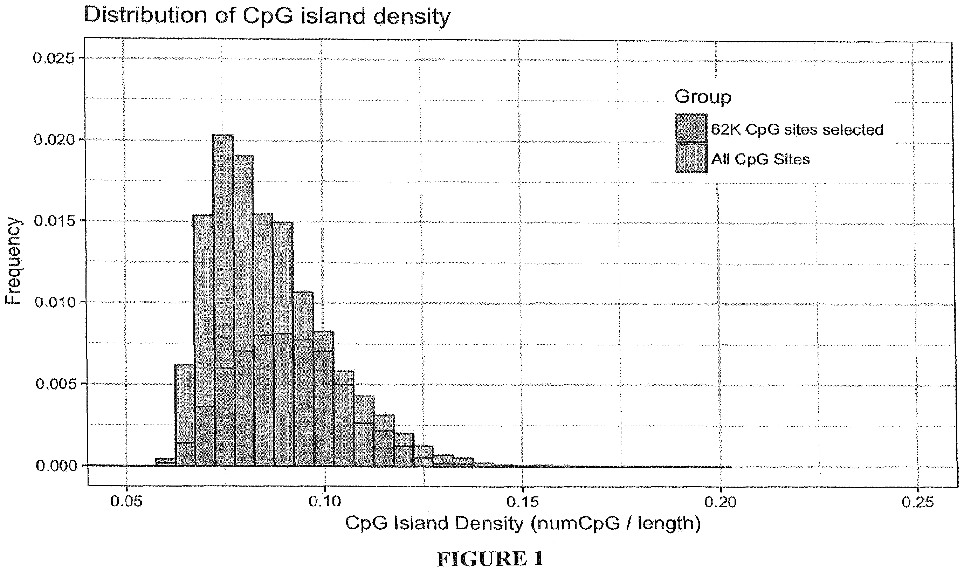

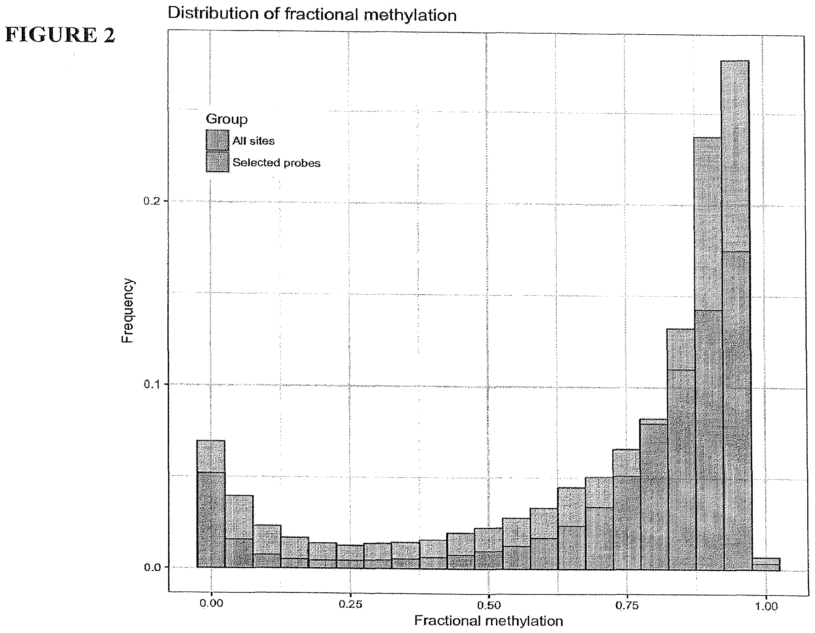

The CpG sites targeted by these probes represent diverse regions of the genome. Within human, 40% of the CpG sites fall within exonic regions, as expected by the known strong conservation signal in exons. The selected set of CpG sites target dense CpG islands due to our choice to include Infinium I probes (Figure 1), and can target both hyper and hypo methylated CpG sites (Figure 2).

### Epigenetic clocks based on highly conserved CpGs in humans

Using 404 highly conserved CpGs on the ILLUMINA 27K array, we developed a novel epigenetic clock using the same data that were previously used for developing the pan-tissue epigenetic clock disclosed in Horvath, S. DNA methylation age of human tissues and cell types, Genome Biol. 14, R115 (2013).

To ensure an unbiased validation in the test data, we only used the training data to define the age predictor. As detailed in Horvath 2013, a transformed version of chronological age was regressed on the CpGs using a penalized regression model (elastic net). The elastic net regression model automatically selected the covariates CpGs. These highly conserved CpGs will be referred to as (epigenetic) clock CpGs since their weighted average (formed by the regression coefficients) amounts to an epigenetic clock. Although the clock was only based on 404 CpGs, the resulting epigenetic age estimator performs remarkably well across a wide spectrum of tissues and cell types (Figure 3).

The linear combination of the 404 highly conserved epigenetic clock CpGs (resulting from the regression coefficients) varies greatly across the entire life course (from cradle to grave) as can be seen from Figure 3. The red calibration curve reveals a logarithmic dependence until adulthood that slows to a linear dependence later in life. The rate of change (of this red curve) can be interpreted as the ticking rate of the epigenetic clock. Similar to the original pan tissue clock from Horvath 2013, we find that organismal growth leads to a high ticking rate that slows down to a constant ticking rate (linear dependence) after adulthood.

### Discussion

The CMAPS algorithm facilitated the design of a novel mammalian methylation array that applies to all mammals. The mammalian array is tailor made for cross species comparisons across mammals and for developing biomarkers that apply to multiple species. Our study demonstrates that relatively few highly conserved CpGs (roughly 400) resulting from CMAPS algorithm already lend themselves for building highly accurate epigenetic age estimators (conserved epigenetic clocks).

Overall, we expect that the mammalian chip is particularly well suited for DNA methylation-based biomarker studies in mammals. For example, the invention allows one to evaluate whether a specific intervention (e.g. a therapeutic agent and/or regimen) that affects DNA methylation levels in one species (e.g. mouse) also affects the corresponding DNA methylation levels in another species (e.g. a human).

### METHODS

### Conserved Methylation Array Probe Selector (CMAPS)

The CMAPS algorithm was applied to the Multiz alignment of 99 vertebrates with the hg19 human genome downloaded from the UCSC Genome Browser (7). For the purpose of this chip, only the mammalian species in this alignment were considered. The design scores for each CpG in the human genome and each possible type of probe at each location were provided by ILLUMINA and taken as input by CMAPS. For each CG site in the human genome, we computed the maximum number of species that could be targeted by each of the 4 different possible probe designs in human, considering each possible placing of the maximum number of tolerated mutations. For each probe option we tried all possibilities for placing the maximum number of potential variants, and greedily picked the variant that covers the most species at a particular position. More specifically, the algorithm for selecting the number of species covered by a probe is explain in pseudocode below:

The function get_max_species makes a greedy choice for the nucleotide at a certain SNV by picking whichever nucleotide is contained by the majority of species in the alignment at that position.

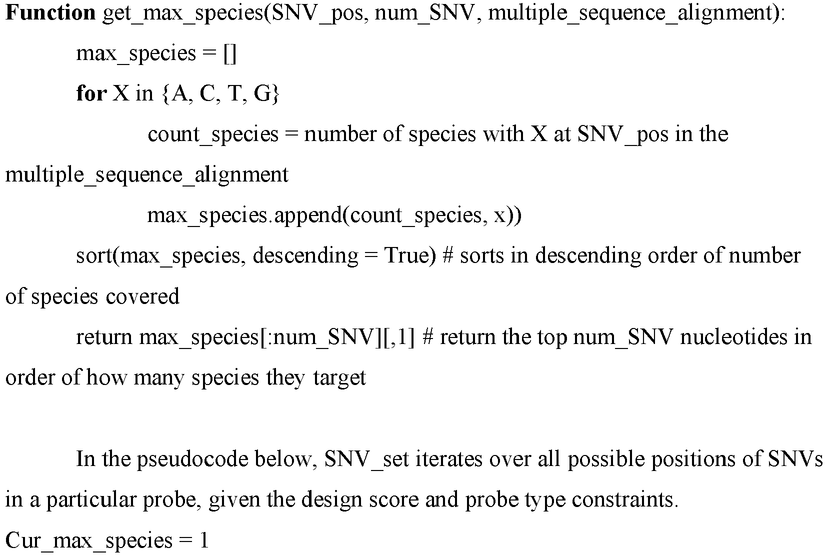

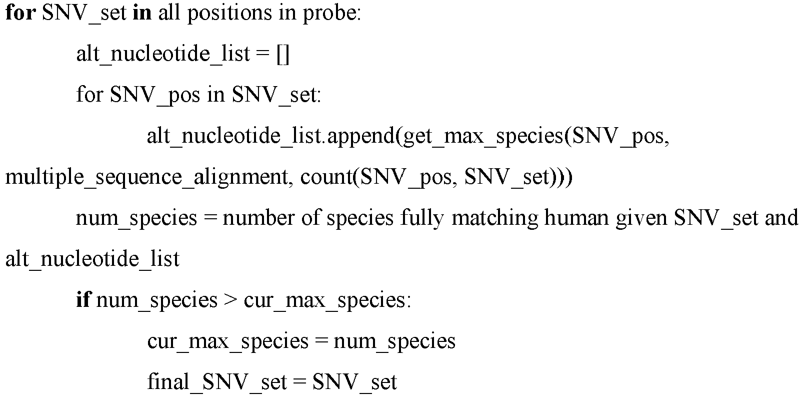

Since the get_max_species function makes greedy choices this may not be the true maximal subset of species for a probe, but this method is relatively computationally inexpensive and produced satisfactory species coverage for our purposes.

### Supplementary Data: SupplementMammalianChip36Kprobes

The following explanation describes the variables.

| | |
|---|---|
| Forward_Sequence: | Sequence on forward strand |
| Genome_Build: | Human Genome build |
| Chromosome: | Human Chromosome CG site is located on |
| Coordinate: | Human Genomic coordinate (1-based) of "C" in the CG site |

| | |
|---|---|
| TB_Strand_OrigP: | TOP/BOTTOM strand |
| Top_Sequence: | Sequence on TOP strand |
| Methyl_Probe_Sequence: | Methylated probe sequence off by one from sequence selected for Infinium 2 |

| | |
|---|---|
| Allele_Fr_Strand: | Forward/Reverse strand |
| Allele_TB_Strand: | TOP/BOT strand |
| Allele_CO_Strand: | Converted/Opposite strand |

| | |
|---|---|
| Underlying_CpG_Count: | Underlying CpG count for each site |
| UnMethyl_Probe_Sequence: | Unmethylated probe sequence off by one from sequence selected for Infinium 2 |
| Num_Species: | Number of mammalian species probe is expected to work in |

Species: Comma separated genome assembly names of the species the probe is expected to work in
Probe_Start_Coord: Probe start coordinate in 1-based hg19 forward strand
Probe_End_Coord: Probe end coordinate in 1-based hg19 forward strand
Reference_Probe_Sequence: Probe forward strand reference sequence in 1-based hg19
SNV_location: hg19 1-based comma separated coordinate of bases where an SNV is designed forSNV_original: hg19 comma separated reference nucleotide for each SNV; 1-1 correspondence with the ordering of coordinates in SNV_location
SNV_change: comma separated alternate designed nucleotide for each SNV; 1-1 correspondence with the ordering of coordiantes in SNV_location and reference nucleotides in SNV_original
Infinium_Type: Inf1/Inf2 Infinium probe type
Is_EPIC_site: 0/1 binary variable indicating whether CG site is also queried by a probe on the EPIC Array
Is_EPIC_design: 0/1 binary variable indicating whether the probe querying this site on the EPIC Array is the same Infinium type(1/2) and same strands(both forward/reverse and converted/opposite); Is always 0 if Is_EPIC_site is 0
Nvariants: Number of variations of the probe based on SNVs effectively 2^(# SNVs) used in mappability analysis

### TABLES

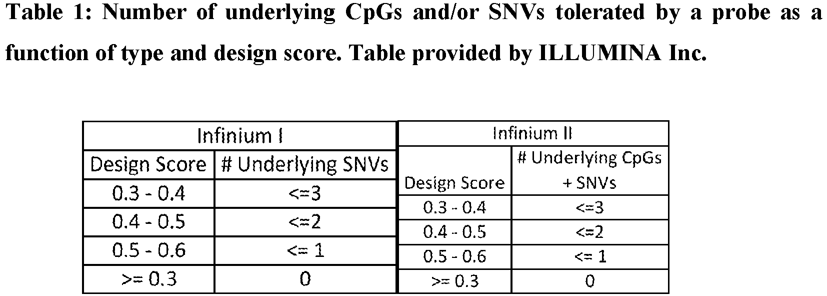

**Table 2. Illustrative Genome/species and the number of applicable probes out of 35,988 probes found by the CMAPS algorithm.**

| **Genome** | **Species** | **No. of Probes** | **Genome** | **Species** | **No. of Probes** |
|---|---|---|---|---|---|
| bosTau7 | Cow | 24817 | musFur1 | Ferret | 25384 |
| calJac3 | Marmoset | 27075 | myoDav1 | David's myotis bat | 19441 |
| camFer1 | Bactrian camel | 23058 | myoLuc2 | Microbat | 19984 |
| canFam3 | Dog | 25305 | nomLeu3 | Gibbon | 30196 |
| capHir1 | Domestic goat | 23913 | ochPri3 | Pika | 16512 |
| cavPor3 | Guinea pig | 18931 | octDeg1 | Brushtailed rat | 19180 |
| cerSim1 | White rhinoceros | 24888 | odoRosDiv1 | Pacific walrus | 26570 |
| chiLan1 | Chinchilla | 21020 | orcOrc1 | Killer whale | 24170 |
| chlSab1 | Green monkey | 32375 | ornAna1 | Platypus | 4867 |
| chrAsi1 | Cape golden mole | 18673 | oryAfel | Aardvark | 20549 |
| conCri1 | Star-nosed mole | 21577 | oryCun2 | Rabbit | 19492 |
| criGri1 | Chinese hamster | 18615 | otoGar3 | Bushbaby | 23249 |
| dasNov3 | Armadillo | 19462 | oviAri3 | Sheep | 24652 |
| echTel2 | Tenrec | 14521 | panHod1 | Tibetan antelope | 24011 |
| eleEdw1 | Cape elephant shrew | 18125 | panTro4 | Chimp | 32809 |
| eptFus1 | Big brown bat | 20555 | papHam1 | Green monkey | 32189 |
| equCab2 | Horse | 23823 | ponAbe2 | Orangutan | 30812 |
| eriEur2 | Hedgehog | 14924 | pteAle1 | Black flying-fox | 23546 |
| felCat5 | Cat | 25252 | pteVam1 | Mega bat | 21250 |
| gorGor3 | Gorilla | 32157 | rheMac3 | Rhesus | 31134 |
| hetGla2 | Naked molerat | 19856 | rn5 | Rat | 18440 |
| jacJac1 | Lesser Egyptian jerboa | 16851 | saiBol1 | Squirrel monkey | 28045 |
| lepWed1 | Weddell seal | 25716 | sarHar1 | Tasmanian devil | 7962 |
| loxAfr3 | Elephant | 19584 | sorAra2 | Shrew | 16776 |
| macEug2 | Wallaby | 6032 | speTri2 | Squirrel | 24393 |
| macFasS | Crab-eating macaque | 32629 | susScr3 | Pig | 22880 |
| mesAur1 | Golden hamster | 18699 | triMan1 | Manatee | 19960 |
| micOch1 | Prairie vole | 18536 | tupChi1 | Chinese tree shrew | 22903 |
| mm10 | Mouse | 22231 | turTru2 | Dolphin | 23396 |
| monDom5 | Opossum | 8160 | vicPac2 | Alpaca | 24455 |

**Table 3: lists illustrative polynucleotide sequences of probes used for querying highly conserved CpGs.**

| **PROBE ID** | **PROBE SEQUENCE** | **SEQ ID NO:** |
|---|---|---|
| cg20254607 | TCCACTGGTACAATTGTCAAATCAATTATTCATTCTCTGCAATTATGCTC | 1 |
| cg13025676 | TGCTCACTTAATTACATGCTTGTTATTGTATTTACACCTTGTTAGATACC | 2 |
| cg24606107 | GTTTTGTAGGAAATGCTATTTATTTTAAATGCTCCACCTGCTGGGAGCCG | 3 |
| cg10304692 | GTCGTAATTTCATGCCCCAATGAGAAGAGCAAGGTCGAAGCAAATGCTTC | 4 |
| cg27662445 | GTAATTTCATGCCCCAATGAGAAGAGCAAGGTCGAAGCAAATGCTTCCAT | 5 |
| cg20141509 | GATCCAATTAATATGCAAATGCAGGAGAGGATTTATTTGTGACATTCTGT | 6 |
| cg25751494 | ACCTAATTAAAAGCTCTGATTGCAGAGATGATTGGGGTAGCGCCAGCAGC | 7 |
| cg18702811 | GAAGGTTACAGGCATCAAAAATTGTTCAGCCGTAATTATTCTTAATGGAT | 8 |
| cg24056059 | GCTTTTTAAATATCCGCTCTGTAATAATGTTTAATTTCAGGGGTCACTCC | 9 |
| cg02958663 | GCTCTGTAATAATGTTTAATTTCAGGGGTCACTCCGCCAAGGAGTATATT | 10 |
| cg01107215 | GGAAAATCAATACCTTTTAAATGCTGTTTATGTGTGATTAACGGTTAATA | 11 |
| cg02551294 | GAAGTTATAATTGATATCGGGGCCCATCACCATAATGGGTTCATCATAGC | 12 |
| cg09531328 | TCAGTCAATACATTTAATAACAACTTACAGGCTATTTTCAATAAAGTGGC | 13 |
| cg19635296 | TGATACAGCAAACACCCAGAGAGATATGATGACAAATGGGTCCAGATCCC | 14 |
| cg13684852 | GAAATTTCATTCAGTTTGTTGCTAGCAGAGATGAAGTAATCTAAATTGTG | 15 |
| cg24978178 | GCAACATTTCTTCTCTGAGCTAATTAAATCTGGAAATGAATTAGCAACAC | 16 |
| cg04108195 | GTTAGTCAATTTAATTTATAATTGAATTGGATGGATGTAACTCTGTGTAA | 17 |
| cg02883001 | TTCATGCGGTAATGACCCTTTTCAGAGACAATGGTCATCATGGATTATGC | 18 |
| cg06637343 | GGTAATGACCCTTTTCAGAGACAATGGTCATCATGGATTATGCGTTTCCA | 19 |
| cg22763089 | GAGGCCTGATCATGTCTGATGGATTGATTTGATTTGCAAATGTAATCAAA | 20 |
| cg22620222 | TCAGAAATTGAAATGGCCCAGATTAATGTATTATATCTTACACACTGTCC | 21 |
| cg18713298 | GAATGTCAACAAAATAAATGAAGTTGCGAGTTGAAGTGAAATTTTTATCA | 22 |
| cg16884658 | GTGAGATTGCTACAGTTCTTGAAGACTTTCCCACAGTACTCACAAGTGTC | 23 |
| cg18295902 | GTTCACAAACTCTTATAGAGTTTTGGAAGTGTGAATCTTTGAAGCCTGAA | 24 |
| cg09680988 | AATTTGCCACTGTTCCACATGATTAAGCCAGATAATTGTGTGTTGATAGC | 25 |
| cg03183633 | GCCTTGGGGTAATGACTAATGTCAATGGCAAATTTCACAGTTGTCTAGAG | 26 |
| cg26441853 | TCCCTGTCTCTGTCATATTTGTCTACTTGAATGGTCCTAAATACCACAGC | 27 |
| cg08271353 | GATTTAGTGGAATGCAATTAGGAAGCCTAAATTAAGTGGTAATGGAGAAC | 28 |
| cg26717373 | ATAAACCTGGCCTCTCTAATCGCCTCCTTATGTGCCTGGAACATCTTGAC | 29 |
| cg02030045 | GCTAAATGTATAGAATCAGCTTCTTGCTAAAAACTACAATTACAGGTGAT | 30 |
| cg07276805 | AACTACAATTACAGGTGATATACAGATTGAAATCACAGGGCTGGTTTGTC | 31 |
| cg03231157 | GGTTGCTGGCCTGAAACAGTATTATTTATATAGAACATTTACGTTTGTTA | 32 |
| cg08816243 | ATTAGTGTCTTGTAATTGTTTCATTAAAACCAGTTGTTCCATTTCTCCTC | 33 |
| cg02231368 | GGCAGAATAATTAATGAATGGTGTCCTTTGTGCTGGTAATAAAGACAAGA | 34 |
| cg25905100 | GAACTTCTTGGAGTTGTTTGCTTTTATAATCAAGGCACAGAAGCAGAACC | 35 |
| cg20217707 | GCTTAGCAGACACTGAAACAAAATGGACTGTAAAGTTCGTTAGATGAAAA | 36 |
| cg20583510 | GTTAGATGAAAATATTAAAAAAGAATTAAGCTAATGGAGATAAAATTAAA | 37 |
| cg04065686 | CACATCACACCAAAAATGGCATTGCAGTGACAGCTAAGATTCCTAATGAC | 38 |
| cg04518473 | TGTCAGCTTCTACCTTGTATGTCCCCAGGCATCAGTAAAATTGACTGCAC | 39 |
| cg23885558 | TAAAGTGCAAATAAAATTTCTAAATTAGAAATTAACACACTCATTCGATC | 40 |
| cg07744194 | ACTCAATTGAAGGTGGCTGTTTCTGAATTAGTCAGCCCTCACAGGCTCTC | 41 |
| cg09684189 | ACTTTTAAATTCTGTACCACCTGTTTTGGGCAAGACATCTTAGGCAGCGC | 42 |
| cg01814663 | TTAAATTCTGTACCACCTGTTTTGGGCAAGACATCTTAGGCAGCGCGACC | 43 |
| cg03273505 | GCCCTGTAGATGTGAAAAAGAAGCAATTATAATGTAGATGAATGATGATT | 44 |
| cg13448855 | CTTACGCTTCTAATTTGTGGCCTTAAATTGCAAACAGAATTTCAGGAGTC | 45 |
| cg25709820 | GTTAAGTACCAGATATTATATTCTGTAATATGCTTAAGTGATATTAGAGG | 46 |
| cg18377384 | GCATAGACCAAAGGTGCTATTAAAGACTGAGTGTATGAAATAGGCAGCAT | 47 |
| cg00010977 | GCCAGTCACAGATATTAAAATGAATTATATCTAATCTGAATTTTAGTCAC | 48 |
| cg04951768 | GCTTGAGAGATAAAACTTTAAGTGTTGCTCCCAATTAGCACAACAGTGAC | 49 |
| cg24868933 | TCAGCATCTGCTTGCATTCAACACAAAATCACTTTGAATTAAAAATTAAC | 50 |

### References Describing methods and materials useful in aspects of the invention

U.S. Patent Publication 20150259742, U.S. Patent App. No. 15/025,185, titled "METHOD TO ESTIMATE THE AGE OF TISSUES AND CELL TYPES BASED ON EPIGENETIC MARKERS", filed by Stefan Horvath; U.S. Patent App. No. 14/119,145, titled "METHOD TO ESTIMATE AGE OF INDIVIDUAL BASED ON EPIGENETIC MARKERS IN BIOLOGICAL SAMPLE", filed by Eric Villain et al.; and Hannum et al. "Genome-Wide Methylation Profiles Reveal Quantitative Views Of Human Aging Rates." Molecular Cell. 2013; 49(2):359-367 and patent US2015/0259742 are relevant to the present invention.

### CITED REFERENCES

1. Bernstein, B. E., Meissner, A. & Lander, E. S. The Mammalian Epigenome. Cell 128, 669-681 (2007).
2. Smith, Z. D. & Meissner, A. DNA methylation: roles in mammalian development. Nature Reviews Genetics 14, 204-220 (2013).
3. Horvath, S. DNA methylation age of human tissues and cell types. Genome Biol. 14, R115 (2013).
4. Genome-wide DNA methylation profiling using Infinium® assay | Epigenomics. Available at:
   https://www.futuremedicine.com/doi/abs/10.2217/epi.09.14. (Accessed: 28th August 2018)
5. Evaluation of the Infinium Methylation 450K technology. - PubMed - NCBI. Available at: https://www.ncbi.nlm.nih.gov/pubmed/22126295. (Accessed: 28th August 2018)
6. Pidsley, R. et al. Critical evaluation of the ILLUMINA MethylationEPIC BeadChip microarray for whole-genome DNA methylation profiling. Genome Biology 17, 208 (2016).
7. Rosenbloom, K. R. et al. The UCSC Genome Browser database: 2015 update. Nucleic Acids Res. 43, D670-681 (2015).

### ADDITIONAL PUBLICATIONS DESCRIBING METHODS AND MATERIALS USEFUL IN ASPECTS OF THE INVENTION

1. Horvath S: DNA methylation age of human tissues and cell types. Genome Biol 2013, 14:R115.
2. Hannum G, Guinney J, Zhao L, Zhang L, Hughes G, Sadda S, Klotzle B, Bibikova M, Fan JB, Gao Y, et al: Genome-wide methylation profiles reveal quantitative views of human aging rates. Mol Cell 2013, 49:359-367.
3. Bocklandt S, Lin W, Sehl ME, Sanchez FJ, Sinsheimer JS, Horvath S, Vilain E: Epigenetic predictor of age. PLoS One 2011, 6:el4821.
4. Levine ME, Lu AT, Quach A, Chen BH, Assimes TL, Bandinelli S, Hou L, Baccarelli AA, Stewart JD, Li Y, et al: An epigenetic biomarker of aging for lifespan and healthspan. Aging (Albany NY) 2018.
5. Zhang Y, Wilson R, Heiss J, Breitling LP, Saum KU, Schottker B, Holleczek B, Waldenberger M, Peters A, Brenner H: DNA methylation signatures in peripheral blood strongly predict all-cause mortality. Nat Commun 2017, 8:14617.
6. Bocklandt S, Lin W, Sehl ME, Sanchez FJ, Sinsheimer JS, Horvath S, Vilain E: Epigenetic predictor of age. PLoS One 2011, 6.
7. Weidner CI: Aging of blood can be tracked by DNA methylation changes at just three CpG sites. Genome Biol 2014, 15.
8. Hannum G: Genome-wide methylation profiles reveal quantitative views of human aging rates. Mol Cell 2013, 49.
9. Lin Q, Weidner CI, Costa IG, Marioni RE, Ferreira MRP, Deary IJ: DNA methylation levels at individual age-associated CpG sites can be indicative for life expectancy. Aging 2016, 8:394-401.
10. Marioni R, Shah S, McRae A, Chen B, Colicino E, Harris S, Gibson J, Henders A, Redmond P, Cox S, et al: DNA methylation age of blood predicts all-cause mortality in later life. Genome Biol 2015, 16:25.
11. Christiansen L, Lenart A, Tan Q, Vaupel JW, Aviv A, McGue M, Christensen K: DNA methylation age is associated with mortality in a longitudinal Danish twin study. Aging Cell 2015.
12. Perna L, Zhang Y, Mons U, Holleczek B, Saum K-U, Brenner H: Epigenetic age acceleration predicts cancer, cardiovascular, and all-cause mortality in a German case cohort. Clinical Epigenetics 2016, 8:1-7.
13. Horvath S, Pirazzini C, Bacalini MG, Gentilini D, Blasio AM, Delledonne M, Mari D, Arosio B, Monti D, Passarino G: Decreased epigenetic age of PBMCs from Italian semi-supercentenarians and their offspring. Aging (Albany NY) 2015, 7.

### CONCLUSION

This concludes the description of the preferred embodiment of the present invention. The foregoing description of one or more embodiments of the invention has been presented for the purposes of illustration and description. It is not intended to be exhaustive or to limit the invention to the precise form disclosed. Many modifications and variations are possible in light of the above teaching.

## Claims

1. A method of making a DNA methylation array comprising a plurality of polynucleotides coupled to a matrix, wherein the plurality of polynucleotides are selected by a method comprising:
(a) performing a polynucleotide sequence alignment comprising comparing a human genome with a plurality of non-human mammalian genomes to identify polynucleotide sequences in the human genome comprising CpG methylation sites that are homologous to polynucleotide sequences within genomes of non-human mammalian species comprising CpG methylation sites;
(b) ranking the polynucleotide sequences in the human genome identified in (a), wherein the ranking criteria comprises sequence homology to polynucleotide sequences in genomes of non-human mammalian species; and
(c) using the ranking in (b) to select a plurality of polynucleotides in the human genome that cross hybridize to a plurality of polynucleotide sequences in the genomes of non-human mammalian species; and
(d) coupling selected sequences from step (c) to a matrix so as to form a DNA methylation array.

2. The method of claim 1, wherein the plurality of human genomic polynucleotide sequences are selected to have not more than a 3 base pair mismatch with polynucleotide sequences in genomes of non-human mammalian species, optionally
wherein the ranking comprises homology comparisons to genomic polynucleotide sequences in non-placental mammalian species, and placental mammalian species in the Laurasiatheria, Euarchontoglires, Xenarthra and Afrotheria superordinal groups, further optionally
wherein the sequence alignment compares human genomic sequences with genomic sequences of at least 10 non-human mammalian species.

3. The method of claim 1, wherein the DNA methylation array comprises at least 30,000 unique polynucleotides coupled to the matrix, optionally
wherein the plurality of unique polynucleotides are between 40-80 nucleotides in length.

4. The method of claim 1, wherein the matrix is a bead or a chip.

5. A method of making a DNA methylation array comprising a plurality of polynucleotides coupled to a matrix, wherein the plurality of polynucleotides:
(a) comprise:
a CpG motif;
at least 2,000 unique polynucleotide sequences that hybridize to a 60 nucleotide segment in genomic polynucleotide sequences of a marsupial mammalian species, a monotreme mammalian species, a Laurasiatheria mammalian species, a Euarchontoglires mammalian species, a Xenarthra mammalian species and an Afrotheria mammalian species with less than a 3 base pair mismatch; and
(b) are selected by:
(i) performing a polynucleotide sequence alignment comparing a human genome with a plurality of non-human mammalian genomes to identify polynucleotide sequences in the human genome comprising CpG methylation sites that are homologous to polynucleotide sequences comprising CpG methylation sites within genomes of non-human mammalian species;
(ii) ranking the polynucleotide sequences in the human genome identified in (a), wherein the ranking criteria comprises a degree of sequence homology to polynucleotide sequences in the genomes of non-human mammalian species; and
(iii) using the ranking in (ii) to select a plurality of polynucleotides having CpG methylation sites that cross hybridize to a plurality of polynucleotide sequences having CpG methylation sites in the genomes of non-human mammalian species with not more than a 3 base pair mismatch; and
(c) coupling selected sequences from step (b) to a matrix so as to form a DNA methylation array;
so that the DNA methylation array is made.

6. A DNA methylation array made by the method of any one of claims 1-5.

7. A DNA methylation array comprising a plurality of polynucleotide sequences coupled to a matrix, wherein:
the polynucleotides comprise at least 40 nucleotides and a CpG motif at their terminal ends;
the polynucleotides comprise polynucleotide sequences present in a human genome; and:
at least 2,000 polynucleotides within the plurality of polynucleotide sequences can hybridize to a 40 nucleotide segment in genomic polynucleotide sequences of a marsupial mammalian species with less than a 3 base pair mismatch;
at least 2,000 polynucleotides within the plurality of polynucleotide sequences can hybridize to a 40 nucleotide segment in genomic polynucleotide sequences of a monotreme mammalian species with less than a 3 base pair mismatch;
at least 2,000 polynucleotides within the plurality of polynucleotide sequences can hybridize to a 40 nucleotide segment in genomic polynucleotide sequences of a Laurasiatheria mammalian species with less than a 3 base pair mismatch;
at least 2,000 polynucleotides within the plurality of polynucleotide sequences can hybridize to a 40 nucleotide segment in genomic polynucleotide sequences of a Euarchontoglires mammalian species with less than a 3 base pair mismatch;
at least 2,000 polynucleotides within the plurality of polynucleotide sequences can hybridize to a 40 nucleotide segment in genomic polynucleotide sequences of a Xenarthra mammalian species with less than a 3 base pair mismatch; and
at least 2,000 polynucleotides within the plurality of polynucleotide sequences can hybridize to a 40 nucleotide segment in genomic polynucleotide sequences of a Afrotheria mammalian species with less than a 3 base pair mismatch.

8. The DNA methylation array of claim 7, wherein:
the marsupial mammalian species is a Wallaby species; and/or
the monotreme mammalian species is a Platypus species; and/or
the Laurasiatheria mammalian species is a bat species; and/or
the Euarchontoglires mammalian species is a rodent species; and/or
the Xenarthra mammalian species is an armadillo species; and/or
the Afrotheria mammalian species is a tenrec species.

9. The DNA methylation array of any one of claims 6--8, wherein at least one polynucleotide within the plurality of polynucleotides is a polynucleotide having a sequence shown in Table 3:
| **PROBE ID** | **PROBE SEQUENCE** | **SEQ ID NO:** |
|---|---|---|
| cg20254607 | TCCACTGGTACAATTGTCAAATCAATTATTCATTCTCTGCAATTATGCTC | 1 |
| cg13025676 | TGCTCACTTAATTACATGCTTGTTATTGTATTTACACCTTGTTAGATACC | 2 |
| cg24606107 | GTTTTGTAGGAAATGCTATTTATTTTAAATGCTCCACCTGCTGGGAGCCG | 3 |
| cg10304692 | GTCGTAATTTCATGCCCCAATGAGAAGAGCAAGGTCGAAGCAAATGCTTC | 4 |
| cg27662445 | GTAATTTCATGCCCCAATGAGAAGAGCAAGGTCGAAGCAAATGCTTCCAT | 5 |
| cg20141509 | GATCCAATTAATATGCAAATGCAGGAGAGGATTTATTTGTGACATTCTGT | 6 |
| cg25751494 | ACCTAATTAAAAGCTCTGATTGCAGAGATGATTGGGGTAGCGCCAGCAGC | 7 |
| cg18702811 | GAAGGTTACAGGCATCAAAAATTGTTCAGCCGTAATTATTCTTAATGGAT | 8 |
| cg24056059 | GCTTTTTAAATATCCGCTCTGTAATAATGTTTAATTTCAGGGGTCACTCC | 9 |
| cg02958663 | GCTCTGTAATAATGTTTAATTTCAGGGGTCACTCCGCCAAGGAGTATATT | 10 |
| cg01107215 | GGAAAATCAATACCTTTTAAATGCTGTTTATGTGTGATTAACGGTTAATA | 11 |
| cg02551294 | GAAGTTATAATTGATATCGGGGCCCATCACCATAATGGGTTCATCATAGC | 12 |
| cg09531328 | TCAGTCAATACATTTAATAACAACTTACAGGCTATTTTCAATAAAGTGGC | 13 |
| cg19635296 | TGATACAGCAAACACCCAGAGAGATATGATGACAAATGGGTCCAGATCCC | 14 |
| cg13684852 | GAAATTTCATTCAGTTTGTTGCTAGCAGAGATGAAGTAATCTAAATTGTG | 15 |
| cg24978178 | GCAACATTTCTTCTCTGAGCTAATTAAATCTGGAAATGAATTAGCAACAC | 16 |
| cg04108195 | GTTAGTCAATTTAATTTATAATTGAATTGGATGGATGTAACTCTGTGTAA | 17 |
| cg02883001 | TTCATGCGGTAATGACCCTTTTCAGAGACAATGGTCATCATGGATTATGC | 18 |
| cg06637343 | GGTAATGACCCTTTTCAGAGACAATGGTCATCATGGATTATGCGTTTCCA | 19 |
| cg22763089 | GAGGCCTGATCATGTCTGATGGATTGATTTGATTTGCAAATGTAATCAAA | 20 |
| cg22620222 | TCAGAAATTGAAATGGCCCAGATTAATGTATTATATCTTACACACTGTCC | 21 |
| cg18713298 | GAATGTCAACAAAATAAATGAAGTTGCGAGTTGAAGTGAAATTTTTATCA | 22 |
| cg16884658 | GTGAGATTGCTACAGTTCTTGAAGACTTTCCCACAGTACTCACAAGTGTC | 23 |
| cg18295902 | GTTCACAAACTCTTATAGAGTTTTGGAAGTGTGAATCTTTGAAGCCTGAA | 24 |
| cg09680988 | AATTTGCCACTGTTCCACATGATTAAGCCAGATAATTGTGTGTTGATAGC | 25 |
| cg03183633 | GCCTTGGGGTAATGACTAATGTCAATGGCAAATTTCACAGTTGTCTAGAG | 26 |
| cg26441853 | TCCCTGTCTCTGTCATATTTGTCTACTTGAATGGTCCTAAATACCACAGC | 27 |
| cg08271353 | GATTTAGTGGAATGCAATTAGGAAGCCTAAATTAAGTGGTAATGGAGAAC | 28 |
| cg26717373 | ATAAACCTGGCCTCTCTAATCGCCTCCTTATGTGCCTGGAACATCTTGAC | 29 |
| cg02030045 | GCTAAATGTATAGAATCAGCTTCTTGCTAAAAACTACAATTACAGGTGAT | 30 |
| cg07276805 | AACTACAATTACAGGTGATATACAGATTGAAATCACAGGGCTGGTTTGTC | 31 |
| cg03231157 | GGTTGCTGGCCTGAAACAGTATTATTTATATAGAACATTTACGTTTGTTA | 32 |
| cg08816243 | ATTAGTGTCTTGTAATTGTTTCATTAAAACCAGTTGTTCCATTTCTCCTC | 33 |
| cg02231368 | GGCAGAATAATTAATGAATGGTGTCCTTTGTGCTGGTAATAAAGACAAGA | 34 |
| cg25905100 | GAACTTCTTGGAGTTGTTTGCTTTTATAATCAAGGCACAGAAGCAGAACC | 35 |
| cg20217707 | GCTTAGCAGACACTGAAACAAAATGGACTGTAAAGTTCGTTAGATGAAAA | 36 |
| cg20583510 | GTTAGATGAAAATATTAAAAAAGAATTAAGCTAATGGAGATAAAATTAAA | 37 |
| cg04065686 | CACATCACACCAAAAATGGCATTGCAGTGACAGCTAAGATTCCTAATGAC | 38 |
| cg04518473 | TGTCAGCTTCTACCTTGTATGTCCCCAGGCATCAGTAAAATTGACTGCAC | 39 |
| cg23885558 | TAAAGTGCAAATAAAATTTCTAAATTAGAAATTAACACACTCATTCGATC | 40 |
| cg07744194 | ACTCAATTGAAGGTGGCTGTTTCTGAATTAGTCAGCCCTCACAGGCTCTC | 41 |
| cg09684189 | ACTTTTAAATTCTGTACCACCTGTTTTGGGCAAGACATCTTAGGCAGCGC | 42 |
| cg01814663 | TTAAATTCTGTACCACCTGTTTTGGGCAAGACATCTTAGGCAGCGCGACC | 43 |
| cg03273505 | GCCCTGTAGATGTGAAAAAGAAGCAATTATAATGTAGATGAATGATGATT | 44 |
| cg13448855 | CTTACGCTTCTAATTTGTGGCCTTAAATTGCAAACAGAATTTCAGGAGTC | 45 |
| cg25709820 | GTTAAGTACCAGATATTATATTCTGTAATATGCTTAAGTGATATTAGAGG | 46 |
| cg18377384 | GCATAGACCAAAGGTGCTATTAAAGACTGAGTGTATGAAATAGGCAGCAT | 47 |
| cg00010977 | GCCAGTCACAGATATTAAAATGAATTATATCTAATCTGAATTTTAGTCAC | 48 |
| cg04951768 | GCTTGAGAGATAAAACTTTAAGTGTTGCTCCCAATTAGCACAACAGTGAC | 49 |
| cg24868933 | TCAGCATCTGCTTGCATTCAACACAAAATCACTTTGAATTAAAAATTAAC | 50 |

10. A method of observing a methylation profile in a non-human mammal comprising:
(a) obtaining genomic DNA from the non-human mammal;
(b) observing cytosine methylation of a plurality CG loci in the genomic DNA using a
DNA methylation array of any one of claims 6-9;
so that a methylation profile in the non-human mammal is observed.

11. The method of claim 10, further comprising:
(c) comparing the CG locus methylation observed in (b) to the CG locus methylation observed in genomic DNA derived from individuals in the non-human mammal species having known ages; and
(d) correlating the CG locus methylation observed in (b) with the known ages of the non-human mammal species;
so that information useful to determine the age of the non-human mammal is obtained.

12. The method of claim 10, wherein:
methylation is observed by a process comprising treatment of genomic DNA from the population of cells from the mammals with bisulfite to transform unmethylated cytosines of CpG dinucleotides in the genomic DNA to uracil;
the DNA methylation array is used to observe methylation profiles in a plurality of non-human mammalian species; and/or
genomic DNA is amplified by a polymerase chain reaction process.

13. A method of observing the effects of a test agent on genomic methylation associated epigenetic aging of mammalian cells, the method comprising:
(a) combining the test agent with mammalian cells;
(b) observing methylation status of methylation markers in genomic DNA from the mammalian cells using a DNA methylation array of any one of claims 6-9;
(c) comparing the observations from (b) with observations of the methylation status in genomic DNA from control mammalian cells not exposed to the test agent such that effects of the test agent on genomic methylation associated epigenetic aging in the mammalian cells is observed.

14. The method of claim 13, wherein one or more of the following:
a) a plurality of test agents are combined with the mammalian cells,
b) the cells are human primary keratinocytes, or
c) the test agent is a compound having a molecular weight less than 3,000, 2,000, 1,000 or 500 g/mol.

15. The method of claim 13, wherein:
methylation is observed by a process comprising treatment of genomic DNA from the population of cells from the mammals with bisulfite to transform unmethylated cytosines of CpG dinucleotides in the genomic DNA to uracil; and/or
genomic DNA is amplified by a polymerase chain reaction process.

## Patentansprüche

1. Ein Verfahren zum Herstellen eines DNA-Methylierungsarrays, das eine Vielzahl von Polynukleotiden beinhaltet, die mit einer Matrix gekoppelt sind, wobei die Vielzahl von Polynukleotiden durch ein Verfahren ausgewählt werden, das Folgendes beinhaltet:
(a) Durchführen eines Polynukleotidsequenzalignments, das das Vergleichen eines menschlichen Genoms mit einer Vielzahl von nicht-menschlichen Säugetiergenomen beinhaltet, um Polynukleotidsequenzen in dem menschlichen Genom zu identifizieren, die CpG-Methylierungsstellen beinhalten, die homolog zu Polynukleotidsequenzen innerhalb von Genomen von nicht-menschlichen Säugetierarten sind, die CpG-Methylierungsstellen beinhalten;
(b) Einordnen der Polynukleotidsequenzen in dem menschlichen Genom, das in (a) identifiziert wird, wobei die Einordnungskriterien Sequenzhomologie zu Polynukleotidsequenzen in Genomen von nicht-menschlichen Säugetierarten beinhalten; und
(c) Verwenden des Einordnens in (b), um eine Vielzahl von Polynukleotiden in dem menschlichen Genom auszuwählen, die zu einer Vielzahl von Polynukleotidsequenzen in den Genomen von nicht-menschlichen Säugetierarten kreuzhybridisieren; und
(d) Koppeln ausgewählter Sequenzen aus Schritt (c) mit einer Matrix, um ein DNA-Methylierungsarray zu bilden.

2. Verfahren gemäß Anspruch 1, wobei die Vielzahl von menschlichen genomischen Polynukleotidsequenzen ausgewählt sind, um nicht mehr als 3 Basenpaarfehlanpassungen mit Polynukleotidsequenzen in Genomen von nicht-menschlichen Säugetierarten aufzuweisen, optional
wobei das Einordnen Homologievergleiche mit genomischen Polynukleotidsequenzen in nicht-plazentaren Säugetierarten und plazentaren Säugetierarten in den übergeordneten Gruppen von Laurasiatheria, Euarchontoglires, Xenarthra und Afrotheria beinhaltet, optional ferner
wobei das Sequenzalignment menschliche genomische Sequenzen mit genomischen Sequenzen von mindestens 10 nicht-menschlichen Säugetierarten vergleicht.

3. Verfahren gemäß Anspruch 1, wobei das DNA-Methylierungsarray mindestens 30 000 eindeutige Polynukleotide, gekoppelt mit der Matrix, beinhaltet, optional wobei die Vielzahl von eindeutigen Polynukleotiden zwischen 40-80 Nukleotide lang sind.

4. Verfahren gemäß Anspruch 1, wobei die Matrix ein Kügelchen oder ein Chip ist.

5. Ein Verfahren zum Herstellen eines DNA-Methylierungsarrays, das eine Vielzahl von Polynukleotiden beinhaltet, die mit einer Matrix gekoppelt sind, wobei die Vielzahl von Polynukleotiden:
(a) Folgendes beinhalten
ein CpG-Motiv;
mindestens 2000 eindeutige Polynukleotidsequenzen, die zu einem 60-Nukleotidsegment in genomischen Polynukleotidsequenzen einer Beuteltier-Säugetierart, einer Kloakentier-Säugetierart, einer Laurasiatheria-Säugetierart, einer Euarchontoglires-Säugetierart, einer Xenarthra-Säugetierart und einer Afrotheria-Säugetierart mit weniger als 3 Basenpaarfehlanpassungen hybridisieren; und
(b) durch Folgendes ausgewählt sind:
(i) Durchführen einer Polynukleotidsequenzalignment, die das Vergleichen eines menschlichen Genoms mit einer Vielzahl von nicht-menschlichen Säugetiergenomen beinhaltet, um Polynukleotidsequenzen in dem menschlichen Genom zu identifizieren, die CpG-Methylierungsstellen beinhalten, die homolog zu Polynukleotidsequenzen sind, die CpG-Methylierungsstellen innerhalb von Genomen von nicht-menschlichen Säugetierarten beinhalten;
(ii) Einordnen der Polynukleotidsequenzen in dem menschlichen Genom, das in (a) identifiziert wird, wobei die Einordnungskriterien einen Grad von Sequenzhomologie zu Polynukleotidsequenzen in den Genomen von nicht-menschlichen Säugetierarten beinhalten; und
(iii) Verwenden des Einordnens in (ii), um eine Vielzahl von Polynukleotiden mit CpG-Methylierungsstellen auszuwählen, die zu einer Vielzahl von Polynukleotidsequenzen mit CpG-Methylierungsstellen in den Genomen von nicht-menschlichen Säugetierarten mit nicht mehr als 3 Basenpaarfehlanpassungen kreuzhybridisieren; und
(c) Koppeln ausgewählter Sequenzen aus Schritt (b) mit einer Matrix, um ein DNA-Methylierungsarray zu bilden;
sodass das DNA-Methylierungsarray hergestellt wird.

6. Ein DNA-Methylierungsarray, das durch das Verfahren gemäß einem der Ansprüche 1-5 hergestellt wird.

7. DNA-Methylierungsarray, das eine Vielzahl von Polynukleotidsequenzen beinhaltet, die mit einer Matrix gekoppelt sind, wobei:
die Polynukleotide mindestens 40 Nukleotide und ein CpG-Motiv an ihren terminalen Enden beinhalten;
die Polynukleotide Polynukleotidsequenzen beinhalten, die in einem menschlichen Genom vorliegen; und
mindestens 2000 Polynukleotide innerhalb der Vielzahl von Polynukleotidsequenzen zu einem 40-Nukleotidsegment in genomischen Polynukleotidsequenzen einer Beuteltier-Säugetierart mit weniger als 3 Basenpaarfehlanpassungen hybridisieren können;
mindestens 2000 Polynukleotide innerhalb der Vielzahl von Polynukleotidsequenzen zu einem 40-Nukleotidsegment in genomischen Polynukleotidsequenzen einer Kloakentier-Säugetierart mit weniger als 3 Basenpaarfehlanpassungen hybridisieren können;
mindestens 2000 Polynukleotide innerhalb der Vielzahl von Polynukleotidsequenzen zu einem 40-Nukleotidsegment in genomischen Polynukleotidsequenzen einer Laurasiatheria-Säugetierart mit weniger als 3 Basenpaarfehlanpassungen hybridisieren können;
mindestens 2000 Polynukleotide innerhalb der Vielzahl von Polynukleotidsequenzen zu einem 40-Nukleotidsegment in genomischen Polynukleotidsequenzen einer Euarchontoglires-Säugetierart mit weniger als 3 Basenpaarfehlanpassungen hybridisieren können;
mindestens 2000 Polynukleotide innerhalb der Vielzahl von Polynukleotidsequenzen zu einem 40-Nukleotidsegment in genomischen Polynukleotidsequenzen einer Xenarthra-Säugetierart mit weniger als 3 Basenpaarfehlanpassungen hybridisieren können; und mindestens 2000 Polynukleotide innerhalb der Vielzahl von Polynukleotidsequenzen zu einem 40-Nukleotidsegment in genomischen Polynukleotidsequenzen einer Afrotheria-Säugetierart mit weniger als 3 Basenpaarfehlanpassungen hybridisieren können.

8. DNA-Methylierungsarray gemäß Anspruch 7, wobei:
die Beuteltier-Säugetierart eine Wallabyart ist; und/oder
die Kloakentier-Säugetierart eine Schnabeltierart ist; und/oder
die Laurasiatheria-Säugetierart eine Fledermausart ist; und/oder
die Euarchontoglires-Säugetierart eine Nagetierart ist; und/oder
die Xenarthra-Säugetierart eine Gürteltierart ist; und/oder
die Afrotheria-Säugetierart eine Tenrekart ist.

9. DNA-Methylierungsarray gemäß einem der Ansprüche 6-8, wobei mindestens ein Polynukleotid innerhalb der Vielzahl von Polynukleotiden ein Polynukleotid mit einer Sequenz ist, die in Tabelle 3 gezeigt ist:

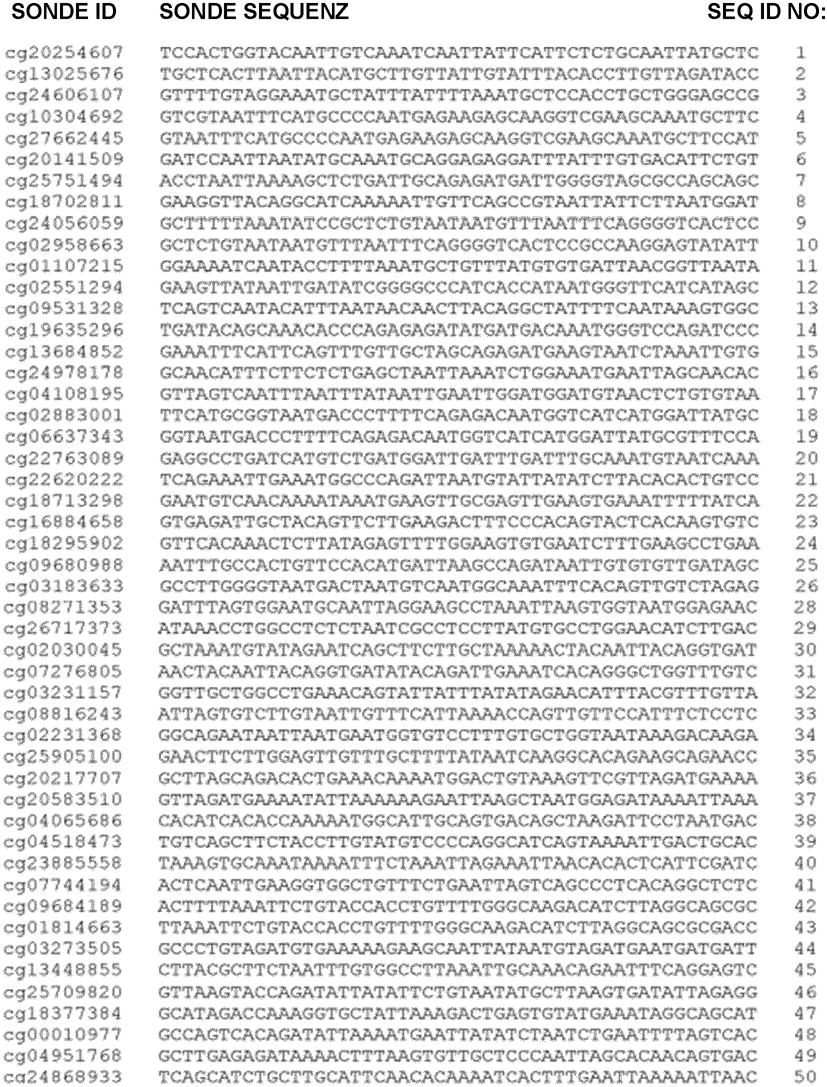

10. Ein Verfahren zum Observieren eines Methylierungsprofils bei einem nicht-menschlichen Säugetier, das Folgendes beinhaltet:
(a) Erhalten von genomischer DNA von dem nicht-menschlichen Säugetier;
(b) Observieren von Cytosinmethylierung einer Vielzahl von OO-Loki in der genomischen DNA unter Verwendung eines DNA-Methylierungsarrays gemäß einem der Ansprüche 6-9;
sodass ein Methylierungsprofil in dem nicht-menschlichen Säugetier observiert wird.

11. Verfahren gemäß Anspruch 10, das ferner Folgendes beinhaltet:
(c) Vergleichen der CG-Locusmethylierung, die in (b) observiert wird, mit der CG-Locusmethylierung, die in genomischer DNA oberviert wird, erlangt von Individuen in der nicht-menschlichen Säugetierart mit bekannten Altern; und
(d) Vergleichen der CG-Locusmethylierung, die in (b) observiert wird, mit den bekannten Altern der nicht-menschlichen Säugetierarten;
sodass Informationen, die nützlich sind, um das Alter des nicht-menschlichen Säugetiers zu bestimmen, erhalten werden.

12. Verfahren gemäß Anspruch 10, wobei:
Methylierung durch einen Prozess beobachtet wird, der eine Behandlung von genomischer DNA von der Population von Zellen von den Säugetieren mit Bisulfit beinhaltet, um unmethylisierte Cytosine von CpG-Dinukleotiden in der genomischen DNA in Uracil zu transformieren;
das DNA-Methylierungsarray verwendet wird, um Methylierungsprofile in einer Vielzahl von nicht-menschlichen Säugetierarten zu observieren; und/oder genomische DNA durch einen Polymerase-Kettenreaktionsprozess amplifiziert wird.

13. Verfahren zum Observieren der Effekte eines Testmittels auf genomische Methylierung, assoziiert mit epigenetischer Alterung von Säugetierzellen, wobei das Verfahren Folgendes beinhaltet:
(a) Kombinieren des Testmittels mit Säugetierzellen;
(b) Observieren des Methylierungsstatus von Methylierungsmarkern in genomischer DNA von den Säugetierzellen unter Verwendung eines DNA-Methylierungsarrays gemäß einem der Ansprüche 6-9;
(c) Vergleichen der Observationen von (b) mit Observationen des Methylierungsstatus in genomischer DNA von Kontrollsäugetierzellen, die nicht dem Testmittel ausgesetzt sind, sodass Effekte des Testmittels auf genomische Methylierung, assoziiert mit epigenetischer Alterung in den Säugetierzellen, observiert werden.

14. Verfahren gemäß Anspruch 13, wobei eines oder mehrere der Folgenden:
a) eine Vielzahl von Testmitteln werden mit den Säugetierzellen kombiniert,
b) die Zellen sind menschliche primäre Keratinozyten, oder
c) das Testmittel ist eine Verbindung mit einem Molekulargewicht von weniger als 3000, 2000, 1000 oder 500 g/mol.

15. Verfahren gemäß Anspruch 13, wobei:
Methylierung durch einen Prozess observiert wird, der die Behandlung von genomischer DNA von der Population von Zellen von den Säugetieren mit Bisulfit beinhaltet, um unmethylisierte Cytosine von CpG-Dinukleotiden in der genomischen DNA in Uracil zu transformieren; und/oder
genomische DNA durch einen Polymerase-Kettenreaktionsprozess amplifiziert wird.

## Revendications

1. Un procédé de fabrication d'un réseau de méthylation d'ADN comprenant une pluralité de polynucléotides couplés à une matrice, la pluralité de polynucléotides étant sélectionnée par un procédé comprenant :
(a) la réalisation d'un alignement de séquences polynucléotidiques comprenant la comparaison d'un génome humain avec une pluralité de génomes mammifères non humains afin d'identifier des séquences polynucléotidiques dans le génome humain comprenant des sites de méthylation CpG qui sont homologues à des séquences polynucléotidiques au sein de génomes d'espèces mammifères non humaines comprenant des sites de méthylation CpG ;
(b) le classement des séquences polynucléotidiques du génome humain identifiées en (a), où les critères de classement comprennent une homologie de séquences avec des séquences polynucléotidiques dans des génomes d'espèces mammifères non humaines ; et
(c) l'utilisation du classement en (b) afin de sélectionner une pluralité de polynucléotides dans le génome humain qui montrent une hybridation croisée avec une pluralité de séquences polynucléotidiques dans les génomes d'espèces mammifères non humaines ; et
(d) le couplage de séquences sélectionnées issues de l'étape (c) à une matrice de manière à former un réseau de méthylation d'ADN.

2. Le procédé de la revendication 1, où la pluralité de séquences polynucléotidiques génomiques humaines sont sélectionnées afin de présenter un mésappariement de pas plus de 3 paires de bases avec des séquences polynucléotidiques dans des génomes d'espèces mammifères non humaines, facultativement
où le classement comprend des comparaisons d'homologie à des séquences polynucléotidiques génomiques dans des espèces mammifères non placentaires, et des espèces mammifères placentaires dans les groupes de super-ordre des Laurasiatheria, Euarchontoglires, Xenarthra et Afrotheria, facultativement encore où l'alignement de séquences compare des séquences génomiques humaines avec des séquences génomiques d'au moins 10 espèces mammifères non humaines.

3. Le procédé de la revendication 1, où le réseau de méthylation d'ADN comprend au moins 30 000 polynucléotides uniques couplés à la matrice, facultativement où la pluralité de polynucléotides uniques font entre 40 et 80 nucléotides de long.

4. Le procédé de la revendication 1, où la matrice est une bille ou une puce.

5. Un procédé de fabrication d'un réseau de méthylation d'ADN comprenant une pluralité de polynucléotides couplés à une matrice, la pluralité de polynucléotides :
(a) comprenant :
un motif CpG ;
au moins 2 000 séquences polynucléotidiques uniques qui s'hybrident à un segment de 60 nucléotides dans des séquences polynucléotidiques génomiques d'une espèce mammifère marsupiale, d'une espèce mammifère monotrème, d'une espèce mammifère des Laurasiatheria, d'une espèce mammifère des Euarchontoglires, d'une espèce mammifère des Xenarthra et d'une espèce mammifère des Afrotheria avec un mésappariement inférieur à 3 paires de bases ; et
(b) étant sélectionnée par :
(i) la réalisation d'un alignement de séquences polynucléotidiques comparant un génome humain avec une pluralité de génomes mammifères non humains afin d'identifier des séquences polynucléotidiques dans le génome humain comprenant des sites de méthylation CpG qui sont homologues à des séquences polynucléotidiques comprenant des sites de méthylation CpG au sein de génomes d'espèces mammifères non humaines ;
(ii) le classement des séquences polynucléotidiques du génome humain identifiées en (a), où les critères de classement comprennent un degré d'homologie de séquences avec des séquences polynucléotidiques dans les génomes d'espèces mammifères non humaines ; et
(iii) l'utilisation du classement en (ii) afin de sélectionner une pluralité de polynucléotides présentant des sites de méthylation CpG qui montrent une hybridation croisée avec une pluralité de séquences polynucléotidiques présentant des sites de méthylation CpG dans les génomes d'espèces mammifères non humaines avec un mésappariement de pas plus de 3 paires de bases ; et
(c) le couplage de séquences sélectionnées issues de l'étape (b) à une matrice de manière à former un réseau de méthylation d'ADN ;
de manière à ce que le réseau de méthylation d'ADN soit fabriqué.

6. Un réseau de méthylation d'ADN fabriqué par le procédé de n'importe laquelle des revendications 1 à 5.

7. Un réseau de méthylation d'ADN comprenant une pluralité de séquences polynucléotidiques couplées à une matrice, où :
les polynucléotides comprennent au moins 40 nucléotides et un motif CpG au niveau de leurs extrémités terminales ;
les polynucléotides comprennent des séquences polynucléotidiques présentes dans un génome humain ; et :
au moins 2 000 polynucléotides au sein de la pluralité de séquences polynucléotidiques peuvent s'hybrider à un segment de 40 nucléotides dans des séquences polynucléotidiques génomiques d'une espèce mammifère marsupiale avec un mésappariement inférieur à 3 paires de bases ;
au moins 2 000 polynucléotides au sein de la pluralité de séquences polynucléotidiques peuvent s'hybrider à un segment de 40 nucléotides dans des séquences polynucléotidiques génomiques d'une espèce mammifère monotrème avec un mésappariement inférieur à 3 paires de bases ;
au moins 2 000 polynucléotides au sein de la pluralité de séquences polynucléotidiques peuvent s'hybrider à un segment de 40 nucléotides dans des séquences polynucléotidiques génomiques d'une espèce mammifère des Laurasiatheria avec un mésappariement inférieur à 3 paires de bases ;
au moins 2 000 polynucléotides au sein de la pluralité de séquences polynucléotidiques peuvent s'hybrider à un segment de 40 nucléotides dans des séquences polynucléotidiques génomiques d'une espèce mammifère des Euarchontoglires avec un mésappariement inférieur à 3 paires de bases ;
au moins 2 000 polynucléotides au sein de la pluralité de séquences polynucléotidiques peuvent s'hybrider à un segment de 40 nucléotides dans des séquences polynucléotidiques génomiques d'une espèce mammifère des Xenarthra avec un mésappariement inférieur à 3 paires de bases ; et
au moins 2 000 polynucléotides au sein de la pluralité de séquences polynucléotidiques peuvent s'hybrider à un segment de 40 nucléotides dans des séquences polynucléotidiques génomiques d'une espèce mammifère des Afrotheria avec un mésappariement inférieur à 3 paires de bases.

8. Le réseau de méthylation d'ADN de la revendication 7, où :
l'espèce mammifère marsupiale est une espèce de wallaby ; et/ou
l'espèce mammifère monotrème est une espèce d'ornithorynque ; et/ou
l'espèce mammifère des Laurasiatheria est une espèce de chauve-souris ; et/ou
l'espèce mammifère des Euarchontoglires est une espèce de rongeur ; et/ou
l'espèce mammifère des Xenarthra est une espèce de tatou ; et/ou
l'espèce mammifère des Afrotheria est une espèce de tenrec.

9. Le réseau de méthylation d'ADN de n'importe laquelle des revendications 6 à 8, où au moins un polynucléotide au sein de la pluralité de polynucléotides est un polynucléotide présentant une séquence montrée dans le Tableau 3 :

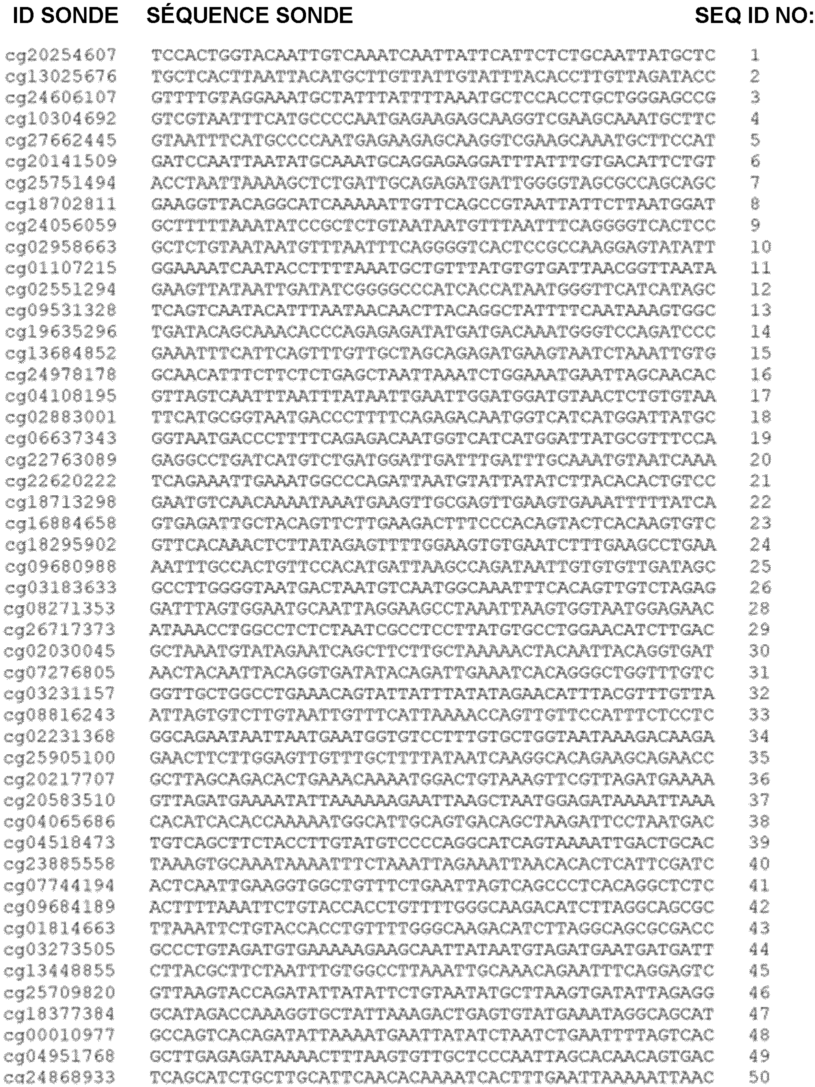

10. Un procédé d'observation d'un profil de méthylation chez un mammifère non humain comprenant :
(a) l'obtention d'ADN génomique issu du mammifère non humain ;
(b) l'observation de la méthylation de cytosine d'une pluralité de locus CG dans l'ADN génomique en utilisant un réseau de méthylation d'ADN de n'importe laquelle des revendications 6 à 9 ;
de manière à ce qu'un profil de méthylation chez le mammifère non humain soit observé.

11. Le procédé de la revendication 10, comprenant en sus :
(c) la comparaison de la méthylation de locus CG observée en (b) à la méthylation de locus CG observée dans de l'ADN génomique dérivé d'individus dans l'espèce mammifère non humaine d'âges connus ; et
(d) la mise en corrélation de la méthylation de locus CG observée en (b) avec les âges connus de l'espèce mammifère non humaine ;
de manière à ce que des informations utiles pour déterminer l'âge du mammifère non humain soient obtenues.

12. Le procédé de la revendication 10, où :
la méthylation est observée par un processus comprenant le traitement de l'ADN génomique issu de la population de cellules provenant des mammifères avec du bisulfite afin de transformer des cytosines non méthylées de dinucléotides CpG dans l'ADN génomique en uracile ;
le réseau de méthylation d'ADN est utilisé pour observer des profils de méthylation dans une pluralité d'espèces mammifères non humaines ; et/ou
l'ADN génomique est amplifié par un processus de réaction en chaîne par polymérase.

13. Un procédé d'observation des effets d'un agent de test sur le vieillissement épigénétique associé à la méthylation génomique de cellules de mammifère, le procédé comprenant :
(a) la combinaison de l'agent de test avec des cellules de mammifère ;
(b) l'observation d'un statut de méthylation de marqueurs de méthylation dans de l'ADN génomique issu des cellules de mammifère en utilisant un réseau de méthylation d'ADN de n'importe laquelle des revendications 6 à 9 ;
(c) la comparaison des observations issues de (b) avec des observations du statut de méthylation dans de l'ADN génomique issu de cellules de mammifère témoins non exposées à l'agent de test de telle sorte que des effets de l'agent de test sur le vieillissement épigénétique associé à la méthylation génomique dans les cellules de mammifère soient observés.

14. Le procédé de la revendication 13, avec une ou plusieurs des caractéristiques suivantes :
a) une pluralité d'agents de test sont combinés avec les cellules de mammifère,
b) les cellules sont des kératinocytes primaires humains, ou
c) l'agent de test est un composé présentant une masse moléculaire inférieure à 3 000, 2 000, 1 000 ou 500 g/mol.

15. Le procédé de la revendication 13, où :
la méthylation est observée par un processus comprenant le traitement de l'ADN génomique issu de la population de cellules provenant des mammifères avec du bisulfite afin de transformer des cytosines non méthylées de dinucléotides CpG dans l'ADN génomique en uracile ; et/ou
de l'ADN génomique est amplifié par un processus de réaction en chaîne par polymérase.
